(19) Europäisches Patentamt
European Patent Office
Office européen des brevets

(11) Numéro de publication: **0 021 924**
**B1**

(12) **FASCICULE DE BREVET EUROPEEN**

(45) Date de publication du fascicule du brevet: **16.03.83**

(51) Int. Cl.³: **C 07 D 401/04,** A 61 K 31/44 // A61K31/40

(21) Numéro de dépôt: **80400808.4**

(22) Date de dépôt: **06.06.80**

(54) **Dérivés de l'indole, procédé pour leur préparation, leur application comme médicaments et compositions pharmaceutiques les contenant.**

(30) Priorité: **12.06.79 FR 7914976**

(43) Date de publication de la demande: **07.01.81 Bulletin 81/1**

(45) Mention de la délivrance du brevet: **16.03.83 Bulletin 83/11**

(84) Etats contractants désignés: **AT BE CH DE FR GB IT LI LU NL SE**

(56) Documents cités:

FR-A-2 391 211
GB-A-1 030 623
GB-A-1 224 530
US-A-3 290 332

CHEMICAL ABSTRACTS, vol. 49, no. 9 10-05-1955, colonne 6225 e-f,Columbus, Ohio, US
H. PLIENINGER: «The alkylation of indole» & Chem. Ber. 87, 127-9 (1954)

(73) Titulaire: **ROUSSEL-UCLAF, 35, boulevard des Invalides, F-75007 Paris (FR)**

(72) Inventeur: **Guillaume, Jacques, 11, allée Hélène Boucher, F-93270 Sevran (FR)**
Inventeur: **Nedelec, Lucien, 45, boulevard de l'Ouest, F-93340 Le Raincy (FR)**
Inventeur: **Dumont, Claude, 33, rue du Maréchal Vaillant, F-94130 Nogent S/Marne (FR)**
Inventeur: **Fournex, Robert, 8, rue du Commandant Rivière, F-75008 Paris (FR)**

(74) Mandataire: **Tonnellier, Marie-José et al, 102, route de Noisy Boîte postale no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 021 924 B1

# Dérivés de l'indole, procédé pour leur preparation, leur application comme médicaments et compositions pharmaceutiques les contenant

La présente invention a pour objet de nouveaux dérivés de l'indole, leur procédé de préparation, leur application comme médicament et les compositions pharmaceutiques les renfermant.

L'invention a pour objet les composés de formule (I):

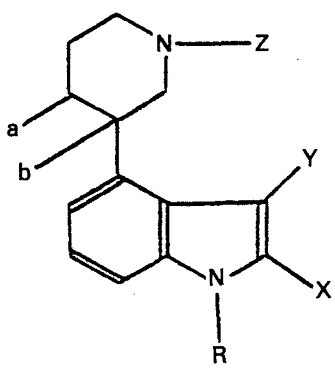

(I)

dans laquelle:

— R représente un atome d'hydrogène, un radical alkyle renfermant de 1 à 8 atomes de carbone, ou un radical aralkyle renfermant de 7 à 12 atomes de carbone,

— X représente un atome d'hydrogène ou un radical alkyle renfermant de 1 à 8 atomes de carbone,

— Y représente un atome d'hydrogène ou un atome d'halogène,

— Z représente un atome d'hydrogène, un radical alkyle ou hydroxyalkyle renfermant de 1 à 8 atomes de carbone, un radical aryloxyalkyle renfermant de 7 à 12 atomes de carbone, un radical aralkyle renfermant de 7 à 12 atomes de carbone, éventuellement substitué par un ou plusieurs atomes d'halogène, par un ou plusieurs radicaux alkoxy renfermant de 1 à 4 atomes de carbone, par un ou plusieurs radicaux alkyles renfermant de 1 à 4 atomes de carbone, ou par un ou plusieurs radicaux OH, $CF_3$, $OCF_3$, $NO_2$ ou $NH_2$, ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone, et

— *ou bien*a et b représentent chacun un atome d'hydrogène,

— *ou bien* a représente un atome d'hydrogène et b représente un radical hydroxyle ou un radical alkoxy renfermant de 1 à 8 atomes de carbone,

— *ou bien* a et b forment ensemble une double liaison carbone-carbone, ainsi que les sels d'addition avec les acides des composés de formule (I).

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxaline, glyoxylique, aspartique, alcanesulfoniques, tels que l'acide méthane sulfonique et aryl sulfoniques, tels que l'acide benzène sulfonique.

Lorsque R représente un radical alkyle, il s'agit de préférence du radical méthyle, éthyle, propyle, isopropyle, butyle, isobutyle ou n-pentyle.

Lorsque R représente un radical aralkyl, il s'agit de préférence du radical benzyle.

Lorsque X représente un radical alkyle, il s'agit de préférence d'un radical alkyle renfermant de 1 à 4 atomes de carbone et notamment du radical méthyle, éthyle, n-propyle et n-butyle.

Lorsque Y représente un atome d'halogène, il s'agit de préférence d'un atome de chlore ou de brome.

Lorsque Z représente un radical alkyle ou hydroxyalkyle, «alkyle» représente de préférence un radical méthyle, éthyle, n-propyle ou isopropyle, n-butyle ou isobutyle, n-pentyle ou n-hexyle.

Lorsque Z représente un radical aryloxyalkyle, il s'agit de préférence du radical phényloxyéthyle ou phényloxypropyle.

Lorsque Z représente un radical aralkyle, on entend de préférence par aralkyle un radical benzyle, phénéthyle ou phénylpropyle.

Lorsque Z représente un radical aralkyle substitué par un ou plusieurs atomes d'halogène, on entend de préférence par halogène un atome de chlore ou de brome.

Lorsque Z représente un radical aralkyle substitué par un ou plusieurs radicaux alkyles, on entend de préférence par alkyle un radical méthyle ou éthyle.

Lorsque Z représente un radical aralkyle substitué par un ou plusieurs radicaux «alkoxy», on entend de préférence par alkoxy un radical méthoxy ou éthoxy.

Lorsque Z représente un radical cycloalkylalkyle, il s'agit de préférence d'un radical cyclopropyle alkyle, par exemple du radical cyclopropylméthyle, cyclopropyléthyle, ou cyclopropyle n-propyle.

Lorsque Z représente un radical alkényle, il s'agit de préférence du radical alkyle.

Lorsque Z représente un radical alkynyle, il s'agit de préférence du radical propargyle.

Lorsque b représente un radical alkoxy, il s'agit de préférence du radical méthoxy ou éthoxy.

Parmi les composés de l'invention, on peut citer, notamment les composés de formule (I) pour lesquels Y représente un atome d'hydrogène ainsi que leurs sels d'addition avec les acides, ceux pour lesquels X représente un atome d'hydrogène ainsi que leurs sels d'addition avec les acides, ceux pour lesquels Z représente un atome d'hydrogène ainsi que leurs sels d'addition avec les acides, ceux pour lesquels Z représente un radical alkyle renfermant de 1 à 4 atomes de carbone ou un radical aralkyle renfermant de 7 à 12 atomes de carbone ainsi que leurs sels d'addition avec les acides. On peut citer encore les composés de formule (I) pour lesquels a et b forment ensemble une double liaison carbone-carbone ainsi que leurs sels d'addition avec les acides, ceux pour lesquels a et b représentent chacun un atome d'hydrogène ainsi que leurs sels d'addition avec les acides, ainsi que ceux pour lesquels a représente un atome d'hydrogène et b un radical hydroxyle ou un radical méthoxy ainsi que leurs sels d'addition avec les acides.

Parmi les composés de l'invention, on peut citer tout particulièrement:

— le 4-(1-propyl 1,2,5,6-tétrahydropyridin-3-yl) 1H-indole,
— le 4-(1-méthyl pipéridin-3-yl) 1H-indole,
— le 4-(1-méthyl 1,2,5,6-tétrahydropyridin-3-yl) 1H-indole
— le 4-(pipéridin-3-yl) 1H-indole,
— le 4-(1,2,5,6-tétrahydropyridin-3-yl) 1H-indole et
— le 4-(1-propyl pipéridin-3-yl) 1H-indole,

ainsi que leurs sels d'addition avec les acides.

Les composés de formule (I) et leurs sels présentent de très intéressantes propriétés pharmacologiques; ils manifestent notamment des propriétés stimulantes dopaminergiques accompagnées ou non d'activités au niveau adrénergique et sérotoninergique.

Ces propriétés permettant d'utiliser les composés de l'invention dans le traitement de très nombreuses maladies ou désordres pathologiques divers; ils peuvent, par exemple, être utilisés dans le traitement des syndromes neurologiques d'origine extrapyramidale, par exemple, dans le traitement de la maladie de Parkinson et dans le traitement des syndromes parkinsoniens post-encéphalitiques; il peuvent être également utilisés dans le traitement de l'hypersecrétion de prolactine par l'antéhypophyse, par exemple, dans le traitement de l'hypogonadisme de la femme ou de l'homme.

Les composés de l'invention peuvent aussi être utilisés dans le traitement de la sénescence cérébrale, de l'insuffisance vertébrobasilaire, de l'hypertension artérielle, des troubles circulatoires périphériques et dans le traitement des artériopathies des membres inférieurs et de leurs complications trophiques.

L'invention a donc pour objet, à titre de médicament, les composés de formule (I) ainsi que leurs sels d'addition avec les acides thérapeutiquement acceptables.

L'invention a tout particulièrement pour objet, à titre de médicament, les composés précédemment nommés, ainsi que leurs sels d'addition avec les acides thérapeutiquement acceptables.

La dose usuelle, variable selon le produit utilisé, le sujet traité et l'affection en cause peut-être, par exemple, de 5 à 100 mg par jour par voie orale chez l'homme pour le traitement de la maladie de Parkinson, ou pour le traitement de la sénescence cérébrale.

L'invention a aussi pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhycules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I) tels que définis précédemment, caractérisé en ce que lon soumet un composés de formule (II):

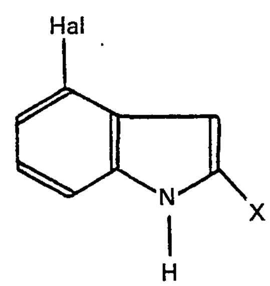

(II)

dans laquelle X représente un atome d'hydrogène ou un radical alkyle renfermant de 1 à 8 atomes de carbone et Hal représente un atome d'halogène, à l'action d'un agent d'alkylation ou d'aralkylation, pour obtenir un composé de formule (III):

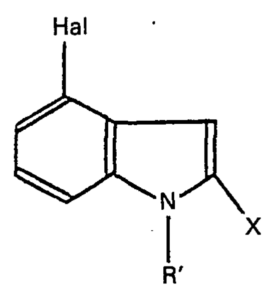

(III)

dans laquelle R' représente un radical alkyle renfermant de 1 à 8 atomes de carbone ou un radical aralkyle renfermant de 7 à 12 atomes de carbone, que l'on transforme en dérivé organomagnésien, pour obtenir un composé de formule (IV):

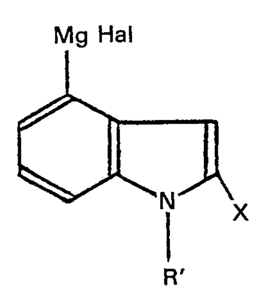

(IV)

que l'on condense avec la N-benzyl 3-pipéridone, pour obtenir un composé de formule ($I_A$):

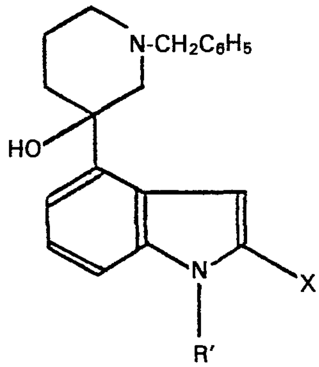

($I_A$)

puis si désiré

— *ou bien l'on soumet le composé de formule ($I_A$)* à l'action d'un agent de déshydratation pour obtenir un composé de formule ($I_B$):

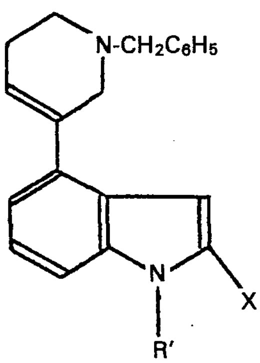

($I_B$)

que l'on soumet si désiré, dans le cas où R' représente un radical benzyle, à l'action d'un agent de clivage sélectif du groupement benzyle porté par le noyau de l'indole pour obtenir un composé de formule ($I_C$):

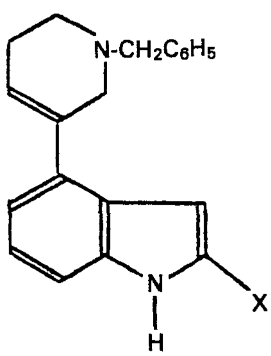

($I_C$)

que l'on soumet, si désiré, à l'action d'un agent de clivage du groupement benzyle porté par le noyau tétrahydropyridinile pour obtenir un composé de formule ($I_D$):

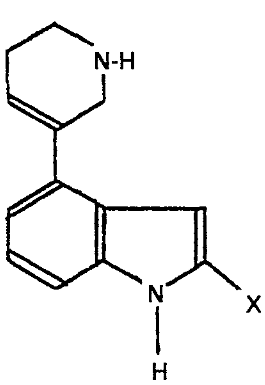

($I_D$)

— *ou bien soumet le composé de formule ($I_A$)* à l'action d'un agent de clivage du groupement benzyle porté par l'azote du groupement pipéridinyle pour obtenir un composé de formule ($I_E$):

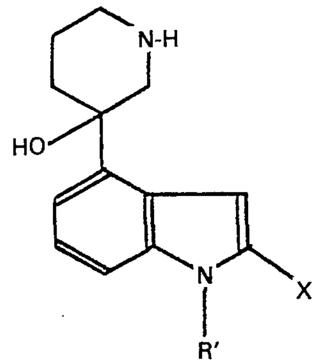

($I_E$)

que l'on soumet, si désiré, à l'action d'un agent de déshydratation pour obtenir un composé de formule ($I_F$):

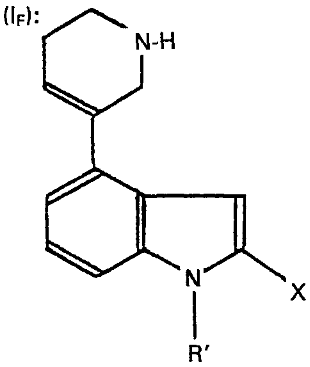

($I_F$)

— *ou bien, dans le cas où R' représente un groupement benzyle, soumet le composé de formule ($I_A$)* à l'action d'un agent de clivage sélectif du groupement benzyle porté par le noyau de l'indole pour obtenir un composé de formule ($I_G$):

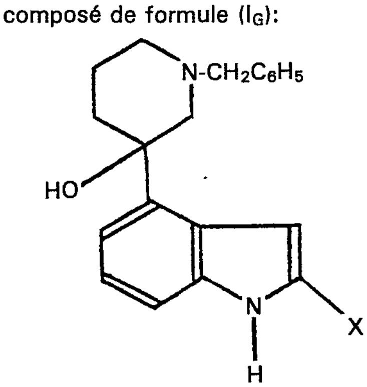

($I_G$)

que l'on soumet, si désiré, à l'action d'un agent de clivage du groupement benzyle porté par le noyau pipéridinyle, pour obtenir un composé de formule ($I_H$):

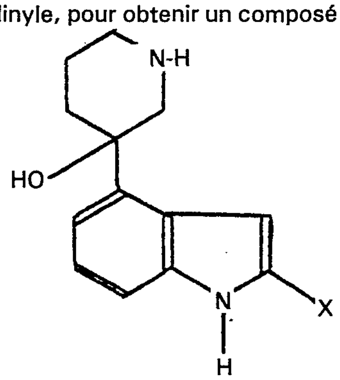

($I_H$)

puis soumet, si désiré, chacun des composés obtenus répondant à la formule (I) dans laquelle Z représente un atome d'hydrogène, à l'action d'un agent capable d'introduire un radical Z', Z' ayant la même signification que Z, à l'exception d'hydrogène, pour obtenir les composés de formule (I) correspondants dans lesquels l'atome d'azote d'un noyau pipéridinyle porte un radical Z', et, si désiré,

— *soit* l'on soumet chacun des composés obtenus répondant à la formule (I) dans laquelle b représente un radical hydroxyle à l'action d'un agent d'éthérification pour obtenir les composés de formule (I) correspondants dans laquelle b représente un radical alkoxy renfermant de 1 à 8 atomes de carbone,

— *soit* l'on soumet chacun des composés obtenus répondant à la formule (I) dans laquelle b représente un radical hydroxyle à l'action d'un agent de déshydratation pour obtenir les composés de formule (I) correspondants dans laquelle a et b forment ensemble une double liaison carbone-carbone,

— *soit* l'on soumet chacun des composés obtenus répondant à la formule (I) dans laquelle b représente un radical hydroxyle à l'action d'un agent de clivage du groupement hydroxyle pour obtenir les composés de formule (I) correspondants dans laquelle a et b représentent chacun un atome d'hydrogène, et, si désiré, soumet chacun des composés de formule (I) ainsi obtenus à l'action d'un agent d'halogénation susceptible d'introduire un atome d'halogène Hal en position 3 de l'indole, pour obtenir le composé de formule (I) correspondants dans lequel Y représente un atome d'halogène,

et, si désiré, soumet chacun des composés de formule (I) obtenus précédemment à l'action d'un acide pour en former le sel.

Il apparaît clairement à l'homme de métier que le procédé de l'invention comporte un certain nombre de stades facultatifs qui peuvent être effectués dans des ordres différents.

L'invention n'est naturellement limitée ni à un nombre de ces stades, ni à un ordre déterminé de ces stades. L'invention s'étend ainsi aux procédés comportant n'importe quel nombre des stades facultatifs indiqués, quelque soit l'ordre dans lequel ces stades sont effectués.

Dans un mode de réalisation préféré du procédé de l'invention:

— on utilise comme produit de départ de formule (II) un produit dans lequel Hal représente un atome de chlore ou de brome;

— l'agent d'alkylation ou d'aralkylation est un halogénure d'alkyle ou d'aralkyle, par exemple, un chlorure, un bromure ou un iodure d'alkyle ou d'aralkyle;

— la formation de magnésien de formule (IV) est réalisée, soit en faisant réagir le magnésium sur le composé de formule (III) dans un solvant approprié, de preférence dans le tétrahydrofurane, en présence d'une petite quantité de dibromoéthane, soit par une réaction d'échange avec un dérivé organométallique comme par exemple le butyllithium;

— l'agent de déshydratation est un acide fort comme l'acide chlorhydrique ou l'acide oxalique ou encore l'anhydride phosphorique;

— l'agent de clivage du groupement benzyle, porté par le noyau de l'indole est le sodium dans l'ammoniac à basse température;

— si a et b ne représentent pas ensemble une double liaison l'agent de clivage du groupement benzyle porté par le noyau pipéridinyle est l'hydrogène en présence d'un catalyseur, par exemple, l'hydrogène en présence de palladium,

— pour cliver le groupement benzyle dans le composé de formule ($I_C$), on fait de préférence réagir le chloroformiate d'éthyle pour former le carbamate d'éthyle correspondant que l'on hydrolyse de préférence en milieu alcalin pour obtenir le composé de formule ($I_D$);

— l'introduction du radical Z' est réalisée au moyen d'un halogénure Z'-Hal dans lequel Hal représente un atome de chlore, de brome ou d'iode;

— l'agent d'éthérification du groupement OH est un alcool en milieu acide anhydre;

— pour effectuer le clivage de groupement OH on utilise le lithium dans l'ammoniac liquide à basse température, par exemple, — 35 à — 60°C;

— l'agent d'halogénation susceptible d'introduire un atome d'halogène en position 3 de l'indole, est un N-halosuccinimide, par exemple le N-chloro ou le N-brome succinimide, dans un solvant approprié, par exemple le dioxane;

— la formation des sels est réalisée selon les méthodes classiques.

Les composés de formule

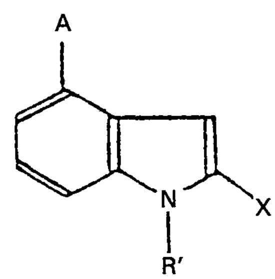

dans laquelle:

— A représente un atome d'halogène Hal, ou un radical MgHal dans lequel Hal représente un atome d'halogène,

— X représente un atome d'hydrogène ou un radical alkyle renfermant de 1 à 8 atomes de carbone,

— R' représente un radical alkyle renfermant de 1 à 8 atomes de carbone, ou un radical aralkyle renfermant de 7 à 12 atomes de carbone,

obtenus lors de la mise en œuvre du procédé de l'invention sont des produits nouveaux.

Les produits de formule (II) utilisés comme produits de départ sont des produits connus de façon générale, ils peuvent être préparés, par exemple, selon le procédé décrit dans Gazz. Chim. Ital. *88*, 1147, (1958).

La N-benzyl 3-pipéridone, également utilisée comme produit de départ est un produit connu, en vente dans le commerce.

Les exemples suivants illustrent l'invention sans toutefois la limiter.

*Exemple 1*

*1-(phényl méthyl) 3-[1-(phényl méthyl)-1H-indole-4-yl] 3-pipéridinol (chlorhydrate)*

Stade A: *4-chloro 1-(phényl méthyl) 1H-indole*

On chauffe à 60°C sous forte agitation, pendant 4 heures, un mélange renfermant 16,9 g de 4-chloro 1H-indole, 170 $cm^3$ de benzène, 85 $cm^3$ d'une solution de lessive de soude à 50%, 1,89 g d'hydrogéno sulfate de n-tétrabutylammonium et 16,6 $cm^3$ de chlorure de benzyle.

On refroidit à la température ambiante, décante, lave la phase organique à l'eau, sèche et chasse les solvants. On obtient 30,2 g d'un produit que l'on chromatographie sur silice en éluant au cyclohexane. On isole ainsi 22,8 g de produit recherché fondant à 58 - 60°C ~.

Stade B: *1-(phényl méthyl) 3-[1-(phényl méthyl) 1H-indol-4-yl] 3-pipéridinol (et chlorhydrate)*

a) *formation du magnésien*

On introduit 113 g de magnésium dans 200 $cm^3$ de tétrahydrofurane. On chauffe au reflux, puis introduit lentement une solution de 184 g de 4-chloro 1-(phényl méthyl) 1H-indole, et de 20 $cm^3$ de 1,2-dibromoéthane dans 300 $cm^3$ de tétrahydrofurane. On maintient le mélange réactionnel au reflux pendant 3 heures et le refroidit à 30°C, On obtient une solution que l'on utilise telle quelle ci-après.

b) *1-(phényl méthyl) 3-[1-(phényl méthyl) 1H-indol-4-yl] 3-pipéridinol (chlorhydrate)*

On introduit dans la solution obtenue au paragraphe a) sans dépasser 35°C, une solution de 128 g de N-benzyl 3-pipéridone dans 250 $cm^3$ de tétrahydrofurane. On chauffe au reflux pendant 2 heures, refroidit à 20°C et introduit lentement 1 litre de chlorure d'ammonium en solution aqueuse saturée. On reprend le mélange réactionnel par 2 litres d'acétate d'éthyle et filtre. On décante, poursuit l'extraction à l'acétate d'éthyle, lave à l'eau, sèche et chasse les solvants. On obtient 330 g d'un produit que l'on chromatographie sur silice (éluant: cyclohexane, triéthylamine 9-1).

On obtient ainsi 218,4 g d'un produit que l'on dissout dans l'acétate d'éthyle. On extrait par de l'acide chlorhydrique 1N. On alcalinise la phase aqueuse acide par l'ammoniaque, et extrait à l'acétate d'éthyle.

On sèche la phase organiqque et ajoute 100 $cm^3$ d'une solution saturée d'acide chlorhydrique dans l'acétate d'éthyle. On filtre le chlorhydrate, lave à l'acétate d'éthyle et à l'éther et sèche.

On obtient 194 g du produit recherché fondant à 185°C. Par recristallisation dans l'acétate d'éthyle à 10% de méthanol, on obtient le produit pur fondant à 190°C.

c) *Libération de la base*

On libère la base correspondante par traitement à la soude et obtient par extraction à l'acétate d'éthyle et évaporation du solvant, le produit recherché.

*Exemple 2*

*Chlorhydrate de 3(1H-indol-4-yl) 1-phényl méthyl-3-pipéridinol*

Stade A: *formation de la base*

On introduit à — 40°C une solution renfermant 14,8 g de 1-(phényl méthyl) 3-[1-(phényl méthyl) 1H-indol-4-yl] 3-pipéridinol dans 250 $cm^3$ de tétrahydrofurane, dans 500 $cm^3$ d'ammoniac. On ajoute progressivement 1,5 g de sodium en maintenant la température à — 40°C. En fin de réaction on ajoute du chlorure d'ammonium jusqu'à décoloration de la teinte bleue obtenue précédemment. On reprend le résidu à l'eau, décante et extrait à l'acétate d'éthyle. On lave à l'eau, sèche et chasse les solvants. On obtient 12 g d'un produit que l'on utilise tel quel dans le stade suivant.

Stade B: *formation de chlorhydrate*

On dissout le produit obtenu au stade A dans l'acétate d'éthyle et ajoute une solution saturée d'acide chlorhydrique dans l'acétate d'éthyle. On filtre, lave à l'acétate d'éthyle et sèche. On obtient 12,7 g de produit recherché fondant à 228-230°C.

*Exemple 3*

*Oxalate de 4-[1-(phényl méthyl) 1,2,5,6-tétrahydropyridin-3-yl] 1H-indole*

Stade A: formation de la base

On chauffe au reflux un mélange de 8 g de chlorhydrate de 3(1H-indol-4-yl) 1-(phényl méthyl) 3-pipéridinol dans 200 $cm^3$ d'acide chlorhydrique 1N. On refroidit à 20-25°C la solution ainsi obtenue, verse le mélange réactionnel dans un mélange d'eau et de glace. On ajoute de l'ammoniaque concentrée et extrait à l'acétate d'éthyle. On sèche, evapore le solvant et obtient 6,35 g d'un produit que l'on chromatographie sur silice en éluant au mélange cyclohexane-triéthylamine (9-1). On isole 4 g du produit recherché.

Stade B: *formation de l'oxalate*

On dissout 3,6 g de produit préparé au stade A dans 200 $cm^3$ d'isopropanol et ajoute 1,5 g d'acide oxalique dihydraté. On porte au reflux en ajoutant du méthanol. On chasse le méthanol sous pression réduite. On obtient un précipité. On refroidit à 0°C, essore et sèche. On obtient ainsi 4,2 g du produit recherché fondant à 202°C.

*Exemple 4*

*3-(1H-indol-4-yl) 3-pipéridinol et chlorhydrate*

Stade A: *Chlorhydrate*

On hydrogène à 60°C, 12,7 g de chlorhydrate de 3-(1H-indol-4-yl) 1-(phényl méthyl) 3-pipéridinol dans 500 $cm^3$ de méthanol en présence de 3,6 g de charbon palladié. On filtre, concentre à sec et obtient 9 g du produit recherché que l'on utilise tel quel pour le stade suivant.

Stade B: *base*

On libère la base correspondante en traitant le produit obtenu au stade A à la soude en solution aqueuse et obtient par extraction à l'acétate d'éthyle le produit recherché.

*Exemple 5*

*oxalate neutre de 3-(1H-indol-4-yl)-1-méthyl--3-pipéridinol*

Stade A: *3-(1H-indol-4-yl)-1-méthyl-3-pipéridinol*

On introduit 13,3 g de 3-(1H-indol-4-yl) 3-pipéridinol dans 120 $cm^3$ de méthanol, puis à 0-5°C, 5,8 g d'une solution de formol à 40% dans l'eau. On agite 15 minutes et ajoute ensuite 4,9 g de borohydrure de sodium à 95%. On ajoute de l'eau et extrait par une solution de chloroforme à 20% de méthanol. On lave à l'eau, sèche sur sulfate de magnésium, filtre et chasse les solvants. On obtient 11,8 g du produit recherché brut que l'on purifie par recristallisation dans le benzène. On obtient ainsi 9 g de produit pur fondant à 145°C.

Stade B: *formation de l'oxalate*

On dissout 4,5 g du produit préparé au stade A dans 500 $cm^3$ d'isopropanol à chaud, puis l'on introduit dans la solution ainsi obtenue 1,2 g d'acide oxalique dihydraté. On ajoute 200 $cm^3$ de méthanol et porte au reflux. On évapore le méthanol. On glace le mélange, essore et sèche les cristaux obtenus. On obtient ainsi 4,95 g du produit recherché fondant à 250°C.

*Exemple 6*

*Oxalate neutre de 4-(1-méthyl 1,2,5,6 -tétrahydropyridin-3-yl) 1H-indole*

Stade A: *formation de la base*

On chauffe au reflux pendant 5 heures une solution renfermant 4,8 g de 1-méthyl 3-(1H-indol-4-yl) 3-pipéridinol dans 150 $cm^3$ d'acide chlorhydrique 1N. On refroidit, dilue à l'eau, ajoute de l'ammoniaque concentrée et extrait au chloroforme à 10% de méthanol. On obtient ainsi 4,4 g d'un produit que l'on chromatographie sur silice en éluant au mélange cyclohexane, chloroforme, triéthylamine (6-3-1). On isole ainsi 2,8 g du produit reherché fondant à 175°C.

Stade B: *formation de l'oxalate*

On dissout 2,4 g du produit préparé au stade A dans 200 $cm^3$ d'isopropanol au reflux. On introduit ensuite 630 mg d'acide oxalique dihydraté. On dissout le précipité obtenu par addition de méthanol. On concentre jusqu'à 100 $cm^3$ environ. On glace le mélange, filtre et obtient ainsi 2,6 g du produit recherché fondant à 232°C.

*Exemple 7*

*4-(1-propyl 1,2,5,6 tétrahydropyridin-3-yl) 1H-indole, fumarate acide*

Stade A: *3-(1H-indol-4-yl) 1-propyl-3-pypéridinol*

On maintient sous agitation à la température ambiante pendant 4 heures une suspension renfermant 6 g de chlorhydrate de 3(1H-indol-4-yl) 3-pipéridinol, 120 $cm^3$ de diméthyl formamide, 7,5 g de carbonate de sodium et 2,8 $cm^3$ d'iodure de propyle. On reprend à l'eau et extrait à l'acétate d'éthyle. On obtient ainsi après élimination du solvant, 5,5 g d'un produit que l'on purifie par chromatographie sur silice en éluant au mélange cyclohexane, chloroforme, triéthylamine (6-3-1). On isole 4,85 g de produit recherché que l'on utilise tel que dans le stade suivant.

Stade B: *fumarate acide de 4-(1-propyl 1,2,5,6 tétrahydropyridin-3-yl) 1H-indole*

a) *formation de la base*

On introduit 4,8 g de 3-(1H-indol-4-yl) 1-propyl 3-pipéridinol dans 150 $cm^3$ d'acide chlorhydrique 1N. On porte le mélange obtenu au reflux pendant 2 heures. On refroidit à la température ambiante, alcalinise et extrait à l'acétate d'éthyle. On sèche sur sulfate de magnésium, filtre et chasse le solvant. On obtient 4,3 g du produit recherché que l'on purifie par chromatographie sur silice en éluant au mélange cyclohexane, chloroforme, triéthylamine (6-3-1). On isole 2,9 g du produit recherché.

b) *formation du fumarate acide*

On dissout 2,7 g de base préparé au stade A dans 200 $cm^3$ d'éthanol et l'on introduit 1,3 g d'acide fumarique. On observe une dissolution de l'acide puis une cristallisation du fumarate. On essore et recristallise le produit obtenu dans l'éthanol et obtient 2,8 g de produit fondant à 168°C.

*Exemple 8*

*3-(1H-indol-4-yl) 1-(2-phényl éthyl) 3-pipéridinol*

On chauffe à 50°C pendant 3 heures une suspension renfermant 6 g de chlorhydrate de 3-(1H-indol-4-yl) 3-pipéridinol, 120 $cm^3$ de diméthylformamide, 7,5 g de carbonate de sodium et 3,9 $cm^3$ de bromure de $\beta$-phényl éthyle. On verse le mélange réactionnel dans l'eau et extrait à l'acétate d'éthyle. On sèche sur sulfate de magnésium, filtre et chasse le solvant. On obtient 8,9 g d'un produit que l'on purifie par chromatographie sur silice en éluant au mélange cyclohexane, chloroforme, triéthylamine (6-3-1). On isole ainsi 6,9 g du produit recherché que l'on utilise dans l'exemple suivant.

*Exemple 9*

*fumarate acide de 4-[1-(2-phényl éthyl) 1,2,5,6--tétrahydropyridin-3-yl] 1H-indole*

Stade A: *formation de la base*

On introduit 6,3 g de 3-(1H-indol-4-yl) 1-(2-phényl éthyl) 3-pipéridinol dans 150 $cm^3$ d'acide chlorhydrique 1N. On chauffe la solution ainsi obtenue pendant 2 h 30. On refroidit à 20-25°C, dilué à l'eau, alcalinise par addition de lessive de soude et extrait à l'acétate d'éthyle. On obtient 6,2 g d'un produit que l'on chromatographie sur silice en éluant au mélange cyclohexane, chloroforme, triéthylamine (6-3-1). On isole ainsi 4,7 g du produit recherché.

Stade B: *fumarate acide du produit préparé au stade A*

On dissout à chaud 4,7 g du produit préparé au stade A dans 100 $cm^3$ d'isopropanol et ajoute 1,8 g d'acide fumarique. Le produit cristallise, on redissout au reflux en ajoutant 200 $cm^3$ d'isopropanol. On concentre ensuite à 200 $cm^3$, glace, essore et obtient ainsi 5,4 g d'un produit que l'on recristallise dans l'acétate d'éthyle à 60% de méthanol. On obtient ainsi 3,9 g du produit recherché fondant à 205°C.

*Exemple 10*

*fumarate neutre de 4-(1-méthyl pipéridin-3-yl) 1H-indole*

Stade A: *formation de la base*

On introduit sous agitation à − 40°C dans 200 $cm^3$ d'ammoniac 40 $cm^3$ de tétrahydrofurane, 20 $cm^3$ d'éthanol anhydre et 5 g de 3-(1H-indol-4-yl)-1-méthyl-3-pipéridinol. On introduit ensuite progressivement par petites fractions toujours à −40 °C 2,6 g de lithium. On maintient encore sous agitation à − 40°C pendant une demi-heure et traite le mélange réactionnel obtenu par addition de chlorure d'ammonium. On laisse évaporer l'ammoniac à la température ambiante, reprend à l'eau et extrait à l'acetate d'éthyle. On obtient 4,65 g d'un produit que l'on chromatographie sur silice en éluant par un mélange chloroforme-méthanol (95-5). On isole ainsi 3 g du produit recherché fondant à 151°C.

Stade B: *formation du fumarate neutre*

On dissout 2,7 g du produit préparé au stade A dans 100 $cm^3$ d'isopropanol et ajoute 800 mg d'acide fumarique. On chauffe au reflux et effectue la dissolution par addition de méthanol. On filtre à chaud, chasse le méthanol sous vide à 40°C, glace, filtre, lave à l'isopropanol et sèche. On obtient ainsi 3 g du produit recherché fondant à 260°C.

*Exemple 11*

*chlorhydrate de 4-(pipéridinyl-3-yl) 1H-indole*

Stade A: *formation de la base*

On introduit à − 40°C dans 100 $cm^3$ d'ammoniac 1,2 g de chlorhydrate de 3-(1H-indol-4-yl) 3-pipéridinol, 20 $cm^3$ de tétrahydrofurane et 10 $cm^3$ d'éthanol anhydre. On introduit ensuite a une heure par petites fractions, 700 mg de lithium. On laisse évaporer l'ammoniac à température ambiante, puis reprend le résidu par 100 $cm^3$ d'eau. On extrait au chloroforme à 10% de méthanol, lave à l'eau et sèche. On filtre et chasse les solvants. On obtient 950 mg d'un produit que l'on chromatographie sur silice (éluant: chloroforme, méthanol, triéthylamine 7-2-1). On isole 665 mg du produit recherché.

Stade B: *formation du chlorhydrate*

On dissout 2,3 g de 4-(pipéridinyl-3-yl) 1H-indole préparé comme il est indiqué au stade A, dans 50 $cm^3$ d'acétate d'éthyle, on ajoute ensuite une solution saturée d'acide chlorhydrique dans l'acétate d'éthyle. On filtre, lave à l'acétate d'éthyle, sèche et recristallise le produit obtenu dans l'acétonitrile à 20% de méthanol. On obtient ainsi 2,2 g du produit recherché.

*Exemple 12*

*oxalate neutre du 4-(1,2,5,6-tétrahydropyridin-3-yl) 1H-indole*

Stade A: *formation de la base*

On chauffe au reflux pendant une heure 7,6 g de 3-(1H-indol-4-yl) 3-pipéridinol dans 300 $cm^3$ d'acide chlorhydrique 1N. On refroidit ensuite à 20 ~ 25°C, ajoute de l'ammoniaque, extrait au chloroforme à 20% de méthanol, et obtient 7 g d'un produit que l'on chromatographie sur silice (éluant: chloroforme, acétone, triéthylamine 6-3-1). On isole 3,4 g de produit que l'on empâte à l'acétone et à l'éther, lave à l'éther et sèche. On obtient 3 g du produit recherché fondant à 156°C.

Stade B: *formation de l'oxalate neutre*

On dissout 3 g de 4-(1,2,5,6-tétrahydropyridin-3-yl) 1H-indole préparé comme il est indiqué au stade A dans 30 $cm^3$ d'isopropanol et ajoute 1,9 g d'acide oxalique dihydraté. On isole 3,8 g du produit attendu que l'on recristallise dans le méthanol. On obtient ainsi 3,3 g de produit pur fondant à 230°C.

*Exemple 13*

*oxalate de 4-(3-méthoxy pipéridin-3-yl) 1H-indole*

Stade A: *4-(3-méthoxy pipéridin-3-yl) 1H-indole*

On dissout 1,5 g de chlorhydrate de 3(1H-indol-4-yl) 3-pipéridinol, dans une solution renfermant 20 $cm^3$ de méthanol et 10 $cm^3$ d'une solution saturée d'acide chlorhydrique dans le méthanol. On maintient la solution obtenue à 20 ~ 25°C pendant 7 heures. On verse le mélange réactionnel dans l'eau et alcalinise par addition de soude.

On filtre, lave à l'eau et sèche le précipité obtenu. On obtient ainsi 900 mg du produit recherché que l'on purifie par recristallisation dans l'acétate d'éthyle. On obtient ainsi le produit recherché fondant à 212°C.

Stade B: *formation de l'oxalate*

On dissout 2,55 g du produit obtenu au stade A dans 200 $cm^3$ d'isopropanol à 40 ~ 50°C. On ajoute ensuite 1,4 g d'acide oxalique dihydraté. On redissout le produit par addition de méthanol. On concentre à 100 $cm^3$, glace, filtre et sèche, pour obtenir 2,7 g du produit recherché fondant a 218°C.

*Exemple 14*

*Chlorhydrate du 1-(phényl méthyl) 4-(1,2,5,6 tétrahydropyridin-3-yl) 1H-indole*

Stade A: *chlorhydrate de 3[1-(phényl méthyl) 1H-indol-4-yl] 3-pipéridinol*

On hydrogène à 40°C 14,4 g de chlorhydrate de 1-(phényl méthyl) 3-[1-(phényl méthyl) 1H-indol-4-yl] 3-pipéridinol (obtenu à l'exemple 1) dans

600 $cm^3$ de méthanol en présence de 4,3 g de noir palladié à 10%. On laisse revenir à température ambiante, filtre le catalyseur, lave au méthanol et chasse les solvants sous pression réduite à 40°C. On obtient 10,65 g de produit attendu Rf : 0,35 (silice - chloroforme - méthanol - triéthylamine 7-2-1).

Stade B: *1-(phényl méthyl) 4-(1,2,5,6-tétrahydropyridin-3-yl) 1H-indole*

On chauffe au reflux pendant 2 heures une solution de 10,4 g de chlorhydrate de 3-[1-(phényl méthyl) 1H-indol-4-yl] 3-pipéridinol dans 500 $cm^3$ d'acide chlorhydrique N. On refroidit, dilue à l'eau, ajoute de la lessive de soude, extrait à l'acétate d'éthyle, évapore et obtient ainsi 7 g d'un résidu que l'on chromatographie sur silice en éluant au mélange cyclohexane, chloroforme, triéthylamine (6-3-1). On isole ainsi 4,7 g du produit recherché sous forme d'une huile brune.

Stade C: *formation du chlorhydrate*

On dissout les 4,7 g du produit préparé au stade B dans 200 $cm^3$ d'acétate d'éthyle, introduit ensuite une solution saturée d'acide chlorhydrique dans l'acétate d'éthyle à 0°C-5°C, filtre, lave à l'acétate d'éthyle, sèche sous vide à 40°C et obtient ainsi 4,7 g du produit recherché fondant à 168°C après recristallisation dans l'isopropanol.

*Exemple 15*

*Oxalate acide de 1-(phényl méthyl) 4-[1-(phényl méthyl) 1,2,5,6-tétrahydropyridin-3-yl] 1H-indole*

Stade A: *1-(phényl méthyl) 4-[1-(phényl méthyl) 1,2,5,6-tétrahydropyridin-3-yl] 1H-indole*

On introduit 53 g de 1-phényl méthyl 3-[1-(phényl méthyl) 1H-indol-4-yl] 3-pipéridinol (obtenu à l'exemple 1), dans 2000 $cm^3$ d'acide chlorhydrique N, chauffe au reflux pendant 7 heures, laisse une nuit à température ambiante, dilue à l'eau, alcalinise à la soude, extrait à l'acétate d'éthyle, après relargage au carbonate de potassium. On lave à l'eau, sèche sur sulfate de magnésium, filtre et chasse les solvants. On obtient 55 g du produit recherché brut que l'on purifie par chromatographie sur silice (éluant: chloroforme-acétone 9-1). On obtient ainsi 31 g de produit purifié sous forme d'une huile brune.

Stade B: *formation de l'oxalate acide*

On dissout 3,6 g du produit préparé au stade A dans 200 $cm^3$ d'éthanol à 40°C puis l'on introduit dans la solution ainsi obtenue 1,2 g d'acide oxalique dihydraté. On filtre à chaud, concentre jusqu'à environ 100 $cm^3$, glace, filtre, lave et sèche les cristaux obtenus. On obtient ainsi 3,8 g du produit recherché fondant à 154°C.

*Exemple 16*

*Succinate neutre de 4-[1-(phényl méthyl) pipéridin-3-yl] 1H-indole*

Stade A: *4-[1-(phényl méthyl) pipéridin-3-yl] 1H-indole*

On agite à — 50°C pendant 2 heures une solution renfermant 33,5 g de 1-phényl méthyl 4-[1-(phényl méthyl) 1,2,5,6-tétrahydropyridin-3-yl] 1H-indole (obtenu à l'exemple 15) dans 300 $cm^3$ de tétrahydrofurane et 500 $cm^3$ d'ammoniac. On introduit ensuite à — 40°C et 8,5 g de sodium, ajoute du chlorure d'ammonium, laisse évaporer l'ammoniac, reprend le résidu à l'eau et extrait à l'acétate d'éthyle. On obtient ainsi 26,80 g d'un produit que l'on chromatographie sur silice en éluant au mélange cyclohexane-chloroforme-triéthylamine (6-3-1). On isole ainsi 22,3 g du produit recherché sous forme d'une huile.

Stade B: *formation du succinate neutre*

On dissout 4 g du produit préparé au Stade A dans 300 $cm^3$ d'isopropanol au reflux. On introduit ensuite 1,6 g d'acide succinique, redissout le précipité formé en ajoutant 200 $cm^3$ d'isopropanol et 200 $cm^3$ de méthanol, filtre à chaud, concentre jusquà 200 $cm^3$ environ. On glace, filtre, lave, sèche sous vide et obtient ainsi 4 g du produit recherché fondant à 210°C.

*Exemple 17*

*Fumarate neutre de 4-(1-propyl-pipéridin-3-yl) 1H-indole*

Stade A: *4-(1-propyl-pipéridin-3-yl) 1H-indole*

On introduit 3,5 g de 4-(pipéridin-3-yl) 1H-indole (préparé à l'exemple 11), dans 70 $cm^3$ de diméthylformamide, puis 3,8 g de carbonate de sodium et 2,2 $cm^3$ d'iodure de propyle, agite pendant 5 heures, précipite à l'eau, extrait à l'acétate d'éthyle, lave la phase organique à l'eau, sèche sur sulfate de magnésium, filtre et chasse le solvant. On obtient 4,1 g du produit recherché brut que l'on purifie par chromatographie sur silice (éluant: cyclohexane-chloroforme, triéthylamine 6-3-1). On obtient ainsi 3,9 g de produit attendu.

Stade B: *formation du fumarate neutre*

On dissout les 3,9 g du produit préparé au stade A dans 200 $cm^3$ d'isopropanol puis l'on introduit dans la solution ainsi obtenue 1 g d'acide fumarique, chauffe au reflux pendant 10 minutes, concentre jusqu'à environ 100 $cm^3$ et amorce la cristallisation. On essore, lave et sèche les cristaux obtenus. On obtient ainsi 3,5 g du produit recherché fondant à 185°C.

*Exemple 18*

*Chlorhydrate du 3-[1-méthyl 1H-indol-4-yl] (phényl méthyl) 3-pipéridinol*

Stade A: *formation du magnésien*

On introduit 12 g de magnésium dans 155 $cm^3$ de tétrahydrofurane, porte au reflux, introduit lentement une solution de 38 g de 1-méthyl 4-chloro 1H-indole et de 4,5 $cm^3$ de 1,2-dibromoéthane dans 115 $cm^3$ de tétrahydrofurane, après amorçage à l'aide de quelques gouttes d'iodure de méthyle,

maintient le reflux pendant 6 heures, refroidit alors à 45°C et obtient une solution que l'on utilise telle quelle ci-après.

Stade B: *chlorhydrate du 3-[1-méthyl 1H-indol-4--yl] 1-(phényl méthyl) 3-pipéridinol*

On introduit goutte à goutte dans la solution obtenue au stade A une solution de 1-benzyl 3-pipéridone préparée à partir de 50 g de chlorhydrate de 1-benzyl-3-pipéridone, dans 115 $cm^3$ de tétrahydrofurane, chauffe 2 heures au reflux, laisse refroidir, agite pendant 16 heures, refroidit au bain de glace, ajoute goutte à goutte 200 $cm^3$ de chlorure d'ammonium en solution saturée, filtre, rince à l'eau et à l'acétate d'éthyle, décante, extrait à nouveau à l'acétate d'éthyle, lave l'eau salée, et distille à sec sous pression réduite. On reprend le résidu à l'éther, extrait à l'acide chlorhydrique N, alcalinise, réextrait à l'acétate d'éthyle et distille à sec sous pression réduite. On obtient 73,4 g de produit brut que l'on purifie par chromatographie sur colonne de silice (éluant: cyclohexane-triéthylamine 9-1). On reprend le produit au chlorure de méthylène, filtre, distille à sec sous pression réduite et obtient 60,5 g de la base du produit attendu.

*Préparation du chlorhydrate*

On dissout le produit obtenu ci-dessus dans 300 $cm^3$ d'acétate d'éthyle, ajoute goutte à goutte une solution d'acide chlorhydrique dans l'acétate d'éthyle jusqu'à pH = 4, glace 16 heures, essore, lave à l'acétate d'éthyle, sèche à 50°C sous vide et obtient 59,6 g de produit attendu F = 250°C.

*Analyse:* $C_{21}$ $H_{25}$ CL $N_2O$ = 356,90

| | | | | |
|---|---|---|---|---|
| Calculé: | C 70,67 | H 7,06 | N 7,85 | Cl 9,93 |
| Trouvé: | C 70,4 | H 6,9 | N 7,9 | Cl 10,1 |

Le 4-chloro 1-méthyl 1H-indole de départ peut être préparé comme suit:

On chauffe à 40°C, sous agitation pendant 4 heures, 60 g de 4-chloro 1H-indole avec 400 $cm^3$ de benzène, 200 $cm^3$ d'une solution de lessive de soude à 50%, 68 g d'hydrogénosulfate de n-tétrabutylammonium et 68 $cm^3$ d'iodure de méthyle, refroidit, décante, réextrait la phase aqueuse à l'acétate d'éthyle, lave les phases organiques réunies à l'eau salée, sèche sur sulfate de magnésium, distille à sec sous pression réduite, purifie par chromatographie sur silice (éluant: cyclohexane-benzène 8-2) et obtient 63,85 g de produit attendu.

*Exemple 19*

*Chlorhydrate de 3-(1-méthyl 1H-indol-4-yl) 3-pipéridinol*

On hydrogène à 40°C 50 g de chlorhydrate de 3-[1-méthyl 1H-indol-4-yl] 1-(phényl méthyl) 3-pipéridinol dans 1,5 litre de méthanol en présence de 15 g de noir palladié. On laisse ensuite refroidir, filtre le catalyseur et distille à sec sous vide. On obtient 37 g du produit attendu. Rf = 0,3, Support : silice - Eluant : chloroforme-méthanol-triéthylamine (6-3-1).

*Exemple 20*

*Oxalate neutre de 1-méthyl 4-(1,2,5,6-tétrahydropyridin-3-yl) 1H-indole*

Stade A: *1-méthyl 4-(1,2,5,6-tétrahydropyridin--3-yl) 1H-indole*

On chauffe au reflux pendant 4 heures une solution renfermant 12 g de chlorhydrate de 3-(1-méthyl 1H-indol-4-yl) 3-pipéridinol dans 360 $cm^3$ d'acide chlorhydrique N. On refroidit au bain de glace, ajoute de la lessive de soude jusqu'à pH alcalin, extrait au chlorure de méthylène, lave à l'eau salée, sèche sur sulfate de magnésium, distille à sec sous pression réduite. On obtient ainsi un produit brut que l'on chromatographie sur silice en éluant au mélange chloroforme-acétone-triéthylamine (6-3-1). On isole ainsi 6 g du produit recherché.

Stade B: *formation de l'oxalate neutre*

On dissout les 6 g du produit préparé au stade A dans 60 $cm^3$ d'isopropanol, introduit ensuite 1,75 g d'acide oxalique en solution dans 35 $cm^3$ d'isopropanol, glace, essore, lave à l'isopropanol, sèche à 50°C sous vide et obtient ainsi 5,4 g du produit recherché fondant à 230°C après recristallisation dans le méthanol.

*Exemple 21*

*3-(1-méthyl 1H-indol-4-yl) 1-propyl 3-pipéridinol*

On introduit 12 g de chlorhydrate de 3-(1-méthyl 1H-indol-4-yl) 3-pipéridinol dans 240 $cm^3$ de diméthylformamide, ajoute 14,5 g de carbonate de sodium et 6 $cm^3$ d'iodure de propyle, agite pendant 20 heures, dilue à l'eau, extrait à l'acétate d'éthyle, lave à l'eau salée, sèche et distille à sec sous pression réduite. On obtient 11 g de produit brut que l'on purifie par chromatographie sur silice (éluant : cyclohexane-triéthylamine 9-1). On obtient ainsi 8,2 g de produit purifié de Rf = 0,2.

*Exemple 22*

*oxalate acide du 1-méthyl 4-(1-propyl 1,2,5,6-tétrahydropyridin-3-yl) 1H-indole*

Stade A: *1-méthyl 4-(1-propyl 1,2,5,6-tétrahydropyridin-3-yl) 1H-indole*

On chauffe au reflux pendant 4 heures une solution renfermant 8,2 g de 3-(1-méthyl 1H-indol-4-yl) 1-propyl 3-pipéridinol dans 250 $cm^3$ d'acide chlorhydrique N. On refroidit au bain de glace, ajoute de la lessive de soude jusqu'à pH alcalin, extrait au chlorure de méthylène, lave à l'eau salée, sèche et distille à sec sous pression réduite. On obtient ainsi un produit brut que l'on chromatographie sur silice en éluant au mélange cyclohexane-triéthylamine (9-1). On isole ainsi 6,2 g du produit recherché de Rf = 0,2.

Stade B: *formation de l'oxalate acide*

On dissout les 6,2 g du produit préparé au stade A dans 62 $cm^3$ d'isopropanol, introduit ensuite 3 g d'acide oxalique en solution dans 30 $cm^3$ d'isopropanol, redissout à chaud le précipité obtenu, amorce la

cristallisation, glace pendant 16 heures, essore, lave à l'isopropanol, sèche sous vide et obtient ainsi 7,5 g du produit recherché fondant à 163°C après recristallisation dans l'éthanol.

*Exemple 23*

*1-méthyl 3-(1-méthyl 1H-indol-4-yl) 3-pipéridinol*

On dissout 16,5 g de chlorhydrate de 3-(1-méthyl 1H-indol-4-yl) 3-pipéridinol dans 20 volumes d'eau, glace et alcalinise par de la soude. On extrait à l'acétate d'éthyle, lave à l'eau, sèche sur sulfate de magnésium et distille à sec sous pression réduite. On dissout le résidu dans 150 $cm^3$ de méthanol, refroidit à 10°C, ajoute 7 $cm^3$ d'une solution de formaldéhyde à 40%, après 15 minutes, refroidit à +5°C et ajoute peu à peu 5 g d'hydroborure de sodium. On agite, après une heure dilue à l'eau et extrait au chlorure de méthylène. On lave à l'eau, sèche et distille à sec sous pression réduite. On obtient 15 g du produit recherché brut fondant à 80°C, qui purifié par chromatographie sur silice (éluant : cyclohexane-triéthylamine 9-1) fond à 90°C.

*Exemple 24*

*Oxalate acide de 1-méthyl 4-(1-méthyl 1,2,5,6-tétrahydropyridin-3-yl) 1H-indole*

Stade A: *1-méthyl 4-(1-méthyl 1,2,5,6-tétrahydropyridin-3-yl) 1H-indole*

On chauffe au reflux pendant 3 heures une solution renfermant 15 g de 1-méthyl 3-(1-méthyl 1H--indol-4-yl) 3-pipéridinol dans 500 $cm^3$ d'acide chlorhydrique N, refroidit à température ambiante, agite pendant 16 heures, refroidit au bain de glace et alcalinise par addition de lessive de soude. On extrait à l'acétate d'éthyle, lave à l'eau salée, sèche sur sulfate de magnésium et distille à sec sous pression réduite. On obtient ainsi 13 g d'un produit que l'on chromatographie sur silice en éluant au mélange cyclohexane-chloroforme-triéthylamine (6-3-1). On isole ainsi 8,7 g du produit recherché.

Stade B: *formation de l'oxalate acide*

On dissout les 8,7 g du produit préparé au stade A dans 87 $cm^3$ d'isopropanol, ajoute une solution de 2,4 g d'acide oxalique dans 48 $cm^3$ d'isopropanol, amorce la cristallisation, glace pendant 16 heures, essore, lave à l'isopropanol, sèche à 50°C sous vide et obtient ainsi 4,3 g du produit recherché fondant à 173°C.

*Exemple 25*

*1-(cyclopropyl méthyl) 3-(1H-indol-4-yl) 3-pipéridinol*

On introduit 14 g de 3-(1H-indol-4-yl) 3-pipéridinol (chlorhydrate) dans 250 $cm^3$ de diméthylformamide, ajoute 17,8 g de carbonate de sodium et 6,4 g de chlorométhylcyclopropane, agite sous atmosphère inerte à 70°C pendant 8 heures, puis 15 heures à température ambiante, reprend le mélange à l'eau, extrait à l'acétate d'éthyle, lave à l'eau, sèche sur sulfate de magnésium, filtre et chasse le solvant à 50°C sous pression réduite. On obtient 13,6 g du produit recherché brut que l'on purifie par chromatographie sur silice (éluant : cyclohexane-chloroforme-triéthylamine 6-3-1). On obtient ainsi 10 g de produit attendu (Rf = 0,15).

*Exemple 26*

*Phosphate de 4-[1-(cyclopropyl méthyl) 1,2,5,6-tétrahydropyridin-3-yl] 1H-indole*

Stade A: *4-[1-(cyclopropyl méthyl) 1,2,5,6-tétrahydropyridin-3-yl] 1H-indole*

On chauffe au reflux pendant 6 heures une solution renfermant 10 g de 1-(cyclopropyl méthyl) 3-(1H-indol-4-yl) 3-pipéridinol dans 300 $cm^3$ d'acide chlorhydrique 1N. On refroidit à température ambiante, ajoute 300 $cm^3$ d'eau, extrait à l'acétate d'éthyle, lave à l'eau salée, sèche et chasse le solvant sous pression réduite. On obtient ainsi 9,1 g d'un produit que l'on chromatographie sur silice en éluant au mélange chloroforme-méthanol (95-5). On isole ainsi 5,4 g du produit recherché (Rf = 0,10).

Stade B: *formation du phosphate*

On dissout 4,9 g du produit préparé au stade A dans 300 $cm^3$ d'isopropanol. On introduit ensuite jusqu'à pH acide une solution d'acide phosphorique à 10% d'isopropanol. On filtre, lave à l'isopropanol et sèche sous vide à 50°C. On recristallise dans un mélange d'éthanol et de méthanol, puis dans l'eau, et obtient 4,5 g du produit recherché fondant à 230°C.

*Exemple 27*

*1-(2-propényl) 3-(1H-indol-4-yl) 3-pipéridinol*

On introduit 18 g de 3-(1H-indol-4-yl) 3-pipéridinol (chlorhydrate) dans 350 $cm^3$ de diméthylformamide, ajoute 22,9 g de carbonate de sodium et 7,6 $cm^3$ de bromure d'allyle puis agite à température ambiante sous atmosphère inerte pendant 2 heures. On reprend le mélange par 1 l d'eau et 500 $cm^3$ d'acétate d'éthyle, décante, lave la phase organique à l'eau, sèche et chasse les solvants sous pression réduite. On obtient 17 g du produit recherché brut que l'on purifie par chromatographie sur silice (éluant : cyclohexane-chloroforme-triéthylamine 6-3-1). On obtient ainsi 14 g de produit purifié (Rf = 0,15).

*Exemple 28*

*Oxalate neutre de 4-[1-(2-propényl) 1,2,5,6-tétrahydropyridin-3-yl] 1H-indole*

Stade A: *4-[1-(2-propényl) 1,2,5,6-tétrahydropyridin-3-yl] 1H-indole*

On chauffe au reflux pendant 4 heures une solution renfermant 14 g de 1-(2-propényl) 3-(1H-indol-4--yl) 3-pipéridinol dans 400 $cm^3$ d'acide chlorhydrique 1N. On refroidit, alcalinise à la lessive de soude, ajoute du carbonate de potassium, extrait à l'acétate d'éthyle, sèche et chasse le solvant sous pression réduite. On obtient ainsi 12,7 g d'un produit que l'on

chromatographie sur silice en éluant au mélange chloroforme-acétone (9-1). On isole ainsi 6,85 g du produit recherché (Rf = 0,15).

Stade B: *formation de l'oxalate neutre*

On dissout 6,8 g du produit preparé au stade A dans 400 $cm^3$ d'isopropanol au reflux. On introduit ensuite 3,6 g d'acide oxalique dihydraté. On maintient au reflux 15 minutes environ. On glace, filtre, sèche et obtient ainsi 6,4 g du produit recherché fondant à 225°C.

*Exemple de compositions pharmaceutiques*

*1) On a réalisé des comprimés répondant à la formule suivante:*

- fumarate neutre de 4-(1-méthyl pipéridin-3-yl) 1H-indole 10 mg;
- excipient (talc, amidon, stéarate de magnésium) q.s.p. pour un comprimé terminé à 150 mg.

2) On a préparé des comprimés répondant à la formule:

- succinate neutre de 4-[1-(phényl méthyl) 1,2,5,6-tétrahydro 3-pyridinyl] 1H-indole 5 mg;
- excipient q.s. pour un comprimé terminé à 100 mg.

(Détail de l'excipient: lactose, amidon, talc, stéarate de magnésium).

3) On a préparé des comprimés répondant à la formule:

- fumarate neutre de 4-(1-propyl 3-pipéridinyl) 1H-indole 2 mg;
- excipient q.s. pour un comprimé terminé à 100 mg.

(Détail de l'excipient: lactose, amidon, talc, stéarate de magnésium).

4) On a préparé des comprimés répondant à la formule:

- oxalate neutre de 4-[1-(2-propényl) 1,2,5,6-tétrahydropyridin-3-yl] 1H-indole 5 mg;
- excipient q.s. pour un comprimé terminé à 100 mg.

(Détail de l'excipient: lactose, amidon, talc, stéarate de magnésium).

*Etude pharmacologique*

1) *Comportement de rotation après lésion unilatérale du faisceau nigrostriatal par la 6-hydroxydopamine - Technique*

La lésion est effectuée chez des rats mâles de 220 g environ par injection unilatérale dans le faisceau dopaminergique nigrostrié, de 8 ug de 6-hydroxydopamine en solution à 2 ug/ul (U. Ungerstedt, Acta Physiol. Scand. 1971, *82*, suppl. 367, 69-93).

Chez de tels animaux, les agonistes dopaminergiques directs, tels que l'apomorphine, administrée par voie générale entraînent un comportement de rotation dans la direction contralatérale au côté lésé.

Le composé étudié est administré aux moins 5 semaines après la lésion. Les animaux sont placée dans un rotomètre automatisé qui permet de compter le nombre de rotations effectuées par chaque animal dans le deux sens.

Dans les conditions de l'expérience, les résultats suivants ont été obtenus:

- les produits des exemples 7 et 10 ont entraîné des rotations contralatérales dès la dose de 2 mg/Kg;
- les produits des exemples 6 et 12 ont entraînédes rotations contralatérales dès la dose de 5 mg/Kg;
- le produit de l'exemple 11 a entraîné des rotations contralatérales dès la dose de 10 mg/Kg;
- les produits des exemples 17 et 28 ont entraîné des rotations contralatérales respectivement dès la dose de 4 et 5 mg/Kg.

Les résultats obtenus montrent que les produits possédent d'intéressantes propriétés stimulantes dopaminergiques.

2) *Détermination de l'activité hypotensive des produits des exemples 6 et 10.*

L'activité hypotensive a été étudiée sur des rats mâles de souche Sprague Dawley S.P.F., pesant 300 g environ et anesthésiés au nembutal (50 mg/Kg par voie intraveineuse).

Le produit testé est administré par voie intraveineuse dans la veine jugulaire.

La pression artérielle carotidienne est mesuré avant et après administration du produit testé.

Il a été constaté que les produits des exemples 6 et 10 présentaient à la dose de 1 mg/Kg une nette activité hypotensive.

3) *Étude de l'anoxie hypobare chez la souris*

On utilise des souris mâles d'un poids de 20 à 22 g, à jeun depuis 5 heures, réparties par groupes de 10 animaux. On détermine le temps de survie des souris placées dans une enceinte hermétique dans laquelle on amène la pression à 90 mm de mercure au moyen d'une pompe. Les produits étudiés sont administrés par voie orale, trente minutes avant l'essai. Les témoins de reçoivent aucun produit.

Les résultats suivants correspondent à l'augmentation du temps de survie exprimés en pourcentage par rapport aux animaux témoins:

| | | |
|---|---|---|
| Produit de l'exemple 6 | 50 mg/Kg | 106% |
| | 10 mg/Kg | 67% |
| | 2 mg/Kg | 30% |
| Produit de l'exemple 7 | 25 mg/Kg | 108% |
| | 5 mg/Kg | 26% |
| Produit de l'exemple 12 | 25 mg/Kg | 44% |

4) *Effet antiagrégant plaquettaire*

L'étude de l'agrégation plaquettaire est effectuée selon la méthode de Born (J. Physiol. 1963, *168*, 178) à l'aide de l'agrégomètre de Mustard.

On utilise des lapins mâles néozélandais. On effectue des prélèvements de sang, par ponction cardiaque. Après centrifugation lente du sang recueilli, on obtient un plasma riche en plaquettes que l'on ajuste à la concentration numérique des 300.000 plaquet-

tes/mm³. On utilise comme agent agrégant le collagène à 40 mcg/ml incubé pendant 5 minutes à 33 °C.

Les produits à étudier sont introduits à des concentrations variables dans le plasma riche en plaquettes.

On mesure la variation de la transmission d'un faisceau lumineux, à travers un tube contenant le plasma riche en plaquettes. Quand les agrégats se forment la quantité de lumière transmise est plus importante et la densité optique diminue.

On détermine l'inhibition de l'agrégation induite par le collagène pour chaque produit à différentes doses.

Les résultats obtenus sont les suivants:

| Produit | Concentration molaire finale | % de diminution de l'agrégation |
|---|---|---|
| Produit de l'exemple 13 | $1 \times 10^{-3}$ | 100% |
| | $1 \times 10^{-4}$ | 42% |
| | $1 \times 10^{-5}$ | 22% |
| Produit de l'exemple 10 | $1 \times 10^{-3}$ | 100% |
| | $1 \times 10^{-4}$ | 76% |
| | $1 \times 10^{-5}$ | 11% |
| Produit de l'exemple 11 | $1 \times 10^{-3}$ | 100% |
| | $1 \times 10^{-4}$ | 100% |
| | $1 \times 10^{-5}$ | 24% |

5) *Étude de la toxicité aiguë*

On a évalué les doses létales $DL_0$ de différents composés après administration par voie intrapéritonéale chez la souris.

On appelle $DL_0$ la dose maximale ne provoquant aucune mortalité.

Les résultats obtenus sont les suivants:

| | | $DL_0$ |
|---|---|---|
| — produit de l'exemple 6 | ≃ | 60 mg/kg |
| — produit de l'exemple 7 | ≃ | 80 — |
| — produit de l'exemple 10 | ≃ | 80 — |
| — produit de l'exemple 11 | ≃ | 60 — |
| — produit de l'exemple 12 | ≃ | 80 — |
| — produit de l'exemple 13 | ≃ | 60 — |
| — produit de l'exemple 17 | ≃ | 40 — |
| — produit de l'exemple 28 | ≃ | 200 — |

**Revendications pour les Etats contractants: BE, CH, LI, DE, FR, GB, IT, LU, NL, SE**

1. Les composés de formule (I):

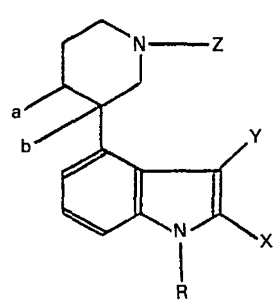

(I)

dans laquelle:

— R représente un atome d'hydrogène, un radical alkyle renfermant de 1 à 8 atomes de carbone, ou un radical aralkyle renfermant de 7 à 12 atomes de carbone,

— X représente un atome d'hydrogène ou un radical alkyle renfermant de 1 à 8 atomes de carbone,

— Y représente un atome d'hydrogène ou un atome d'halogène,

— Z représente un atome d'hydrogène, un radical alkyle ou hydroxyalkyle renfermant de 1 à 8 atomes de carbone, un radical aryloxyalkyle renfermant de 7 à 12 atomes de carbone, un radical aralkyle renfermant de 7 à 12 atomes de carbone éventuellement substitué par un ou plusieurs atomes d'halogène, par un ou plusieurs radicaux alkoxy renfermant de 1 à 4 atomes de carbone, par un ou plusieurus radicaux alkyles renfermant de 1 à 4 atomes de carbone, ou par un ou plusieurs radicaux OH, $CF_3$, $OCF_3$, $NO_2$ ou $NH_2$ ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone et,

— ou bien a et b représentent chacun un atome d'hydrogène,

— ou bien a représente un atome d'hydrogène et b représente un radical hydroxyle ou un radical alkoxy renfermant de 1 à 8 atomes de carbone,

— ou bien a et b forment ensemble une double liaison carbone-carbone ainsi que les sels d'addition avec les acides des composés de formule (I).

2. Les composés de formule (I) tels que définis à la revendication 1, pour lesquels Y représente un atome d'hydrogène, ainsi que leurs sels d'addition avec les acides.

3. Les composés de formule (I) tels que définis à la revendication 1 ou 2, pour lesquels X représente un atome d'hydrogène, ainsi que leurs sels d'addition avec les acides.

4. Les composés de formule (I) tels que définis à la revendication 1, 2 ou 3, pour lesquels Z représente un atome d'hydrogène, ainsi que leurs sels d'addition avec les acides.

5. Les composés de formule (I) tels que définis à l'une quelconque des revendications 1, 2 ou 3, pour lesquels Z représente un radical alkyle renfermant de 1 à 4 atomes de carbone ou un radical aralkyle renfermant de 7 à 12 atomes de carbone, ainsi que leurs sels d'addition avec les acides.

6. Les composés de formule (I) tels que définis à l'une quelconque des revendications 1 à 5, pour lesquels a et b forment ensemble une double liaison carbone-carbone, ainsi que leurs sels d'addition avec les acides.

7. Les composés de formule (I) tels que définis à l'une quelconque des revendications 1 à 5, pour lesquels a et b représentent chacun un atome d'hydrogène, ainsi que leurs sels d'addition avec les acides.

8. Les composés de formule (I) tels que définis à l'une quelconque des revendications 1 à 5, pour lesquels a représente un atome d'hydrogène et b un radical hydroxyle ou un radical méthoxy ainsi que leurs sels d'addition avec les acides.

9. Le 4-(pipéridin-3-yl) 1H-indole ainsi que ses sels d'addition avec les acides.

10. Le 4-(1,2,5,6-tétrahydropyridin-3-yl) 1H-indole ainsi que ses sels d'addition avec les acides.

11. Procédé de préparation des composés de formule (I) tels que définis à l'une quelconque des revendications 1 à 10, caractérisé en ce que l'on soumet un composé de formule (II)

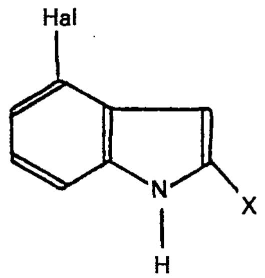

(II)

dans laquelle X représente un atome d'hydrogène ou un radical alkyle renfermant de 1 à 8 atomes de carbone et Hal représente un atome d'halogène, à l'action d'un agent d'alkylation ou d'aralkylation, pour obtenir un composé de formule (III):

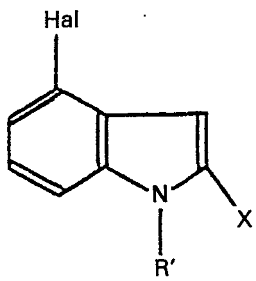

(III)

dans laquelle R' représente un radical alkyle renfermant de 1 à 8 atomes de carbone ou un radical aralkyle renfermant de 7 à 12 atomes de carbone, que l'on transforme en dérivé organo-magnésien, pour obtenir un composé de formule (IV):

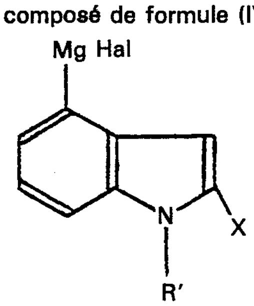

(IV)

que l'on condense avec la N-benzyl 3-pipéridone, pour obtenir un composé de formule ($I_A$):

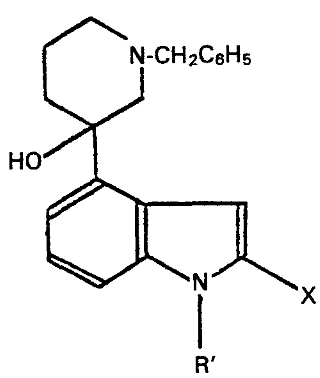

($I_A$)

puis, si désiré,

— *ou bien* soumet le composé de formule ($I_A$), à l'action d'un agent de déshydratation, pour obtenir un composé de formule ($I_B$):

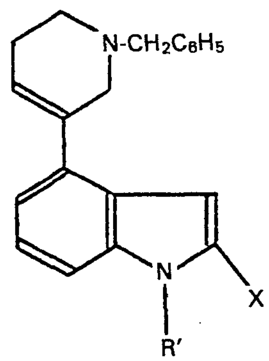

($I_B$)

que l'on soumet, si désiré, dans le cas où R' représente un radical benzyle, à l'action d'un agent de clivage sélectif du groupement benzyle porté par le noyau de l'indole, pour obtenir un composé de formule ($I_C$):

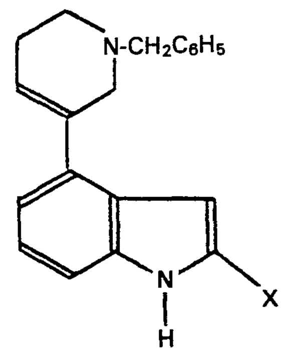

($I_C$)

que l'on soumet, si désiré, à l'action d'un agent de clivage du groupement benzyle porté par le noyau tétrahydropyridinyle, pour obtenir le composé de formule ($I_D$):

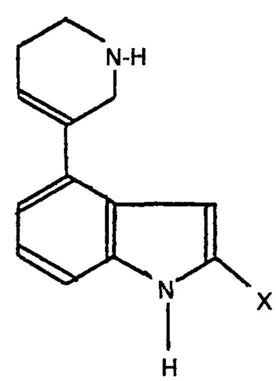

($I_D$)

— *ou bien* soumet le composé de formule ($I_A$), à l'action d'un agent de clivage du groupement benzyle porté par l'azote du groupement pipéridinyle, pour obtenir un composé de formule ($I_E$):

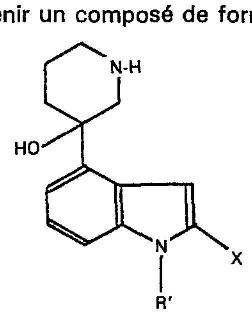

($I_E$)

que l'on soumet, si désiré, à l'action d'un agent de déshydratation, pour obtenir un composé de formule ($I_F$):

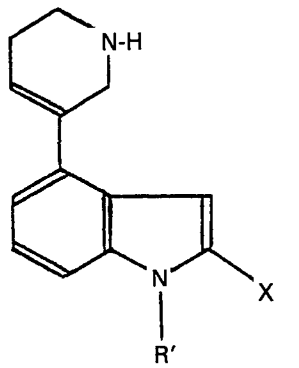

($I_F$)

— *ou bien,* dans le cas où R' représente un groupement benzyle soumet le composé de formule ($I_A$), à l'action d'un agent de clivage sélectif du groupement benzyle, soumet le composé de formule ($I_A$), tenir un composé de formule ($I_G$):

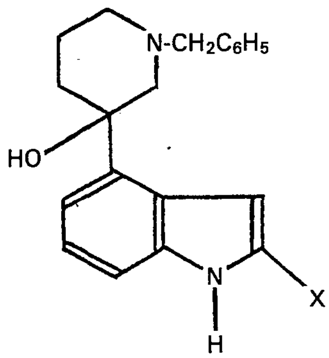

($I_G$)

que l'on soumet, si désiré, à l'action d'un agent de clivage du groupement benzyle porté par le noyau pipéridinyle, pour obtenir un composé de formule ($I_H$):

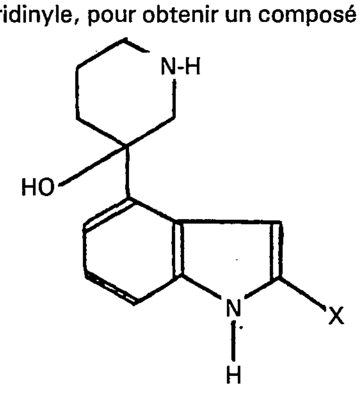

($I_H$)

puis soumet, si désiré, chacun des composés obtenus répondant à la formule (I) dans laquelle Z représente un atome d'hydrogène à l'action d'un agent capable d'introduire un radical Z', Z' ayant la même signification que Z, enoncée à la revendication 1, à l'exception d'hydrogène, pour obtenir les composés de formule (I) correspondants dans lesquels l'atome d'azote du noyau pipéridinyle porte un radical Z' et, si désiré,

— soit l'on soumet chacun des composés obtenus répondant à la formule (I) dans laquelle b représente un radical hydroxyle à l'action d'un agent d'éthérification, pour obtenir les composés de formule (I) correspondants dans laquelle b représente un radical alkoxy renfermant de 1 à 8 atomes de carbone,

— soit l'on soumet chacun des composés obtenus répondant à la formule (I) dans laquelle b représente un radical hydroxyle à l'action d'un agent de déshydratation, pour obtenir les composés de formule (I) correspondants dans laquelle a et b forment ensemble une double liaison carbone-carbone,

— soit l'on soumet chacun des composés obtenus répondant à la formule (I) dans laquelle b représente un radical hydroxyle à l'action d'un agent de clivage du groupement hydroxyle, pour obtenir les composés de formule (I) correspondants dans laquelle a et b représentent chacun un atome d'hydrogène et, si désiré, soumet chacun des composés de formule (I) ainsi obtenus à l'action d'un agent d'halogénation susceptible d'introduire un atome d'halogène Hal en position 3 de l'indole, pour obtenir le composé de formule (I) correspondant dans lequel Y représente un atome d'halogène, et, si désiré, soumet chacun des composés de formule (I) obtenus précédemment à l'action d'un acide pour en former le sel.

12. Procédé selon la revendication 11, caractérisé en ce que les différentes étapes facultatives sont réalisées dans quelqu'ordre que ce soit.

13. A titre de médicaments, les composés de formule (I), tels que définis à l'une quelconque des revendications 1 à 8, ainsi que leurs sels d'addition avec les acides pharmaceutiquement acceptables.

14. A titre de médicaments, les composés de formule (I), tels que définis à l'une des revendications 9 ou 10, pharmaceutiquement acceptables.

15. A titre de médicament:

— le chlorhydrate de 4-(pipéridin-3-yl) 1H-indole.

16. Les compositions pharmaceutiques renfermant comme principe actif, l'un au moins des médicaments tels que définis à la revendication 13, 14 ou 15.

**Revendications pour l'Etat contractant: AT**

1. Procédé de préparation des composés de formule (I):

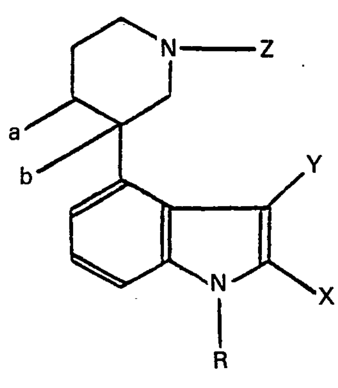

(I)

dans laquelle:

— R représente un atome d'hydrogène, un radical alkyle renfermant de 1 à 8 atomes de carbone, ou un radical aralkyle renfermant de 7 à 12 atomes de carbone,

— X représente un atome d'hydrogène ou un radical alkyle renfermant de 1 à 8 atomes de carbone,

— Y représente un atome d'hydrogène ou un atome d'halogène,

— Z représente un atome d'hydrogène, un radical

alkyle ou hydroxyalkyle renfermant de 1 à 8 atomes de carbone, un radical aryloxyalkyle renfermant de 7 à 12 atomes de carbone, un radical aralkyle renfermant de 7 à 12 atomes de carbone éventuellement substitué par un ou plusieurs atomes d'halogène, par un ou plusieurs radicaux alkoxy renfermant de 1 à 4 atomes de carbone, par un ou plusieurs radicaux alkyles renfermant de 1 à 4 atomes de carbone, ou par un ou plusieurs radicaux OH, $CF_3$, $OCF_3$, $NO_2$ ou $NH_2$ ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone et,

— ou bien a et b représentent chacun un atome d'hydrogène,

— ou bien a représente un atome d'hydrogène et b représente un radical hydroxyle ou un radical alkoxy renfermant de 1 à 8 atomes de carbone,

— ou bien a et b forment ensemble une double liaison carbone-carbone ainsi que de leurs sels d'addition avec les acides, caractérisé en ce que l'on soumet un composé de formule (II):

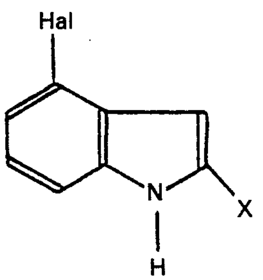

(II)

dans laquelle X représente un atome d'hydrogène ou un radical alkyle renfermant de 1 à 8 atomes de carbone et Hal représente un atome d'halogène, à l'action d'un agent d'alkylation ou d'aralkylation, pour obtenir un composé de formule (III):

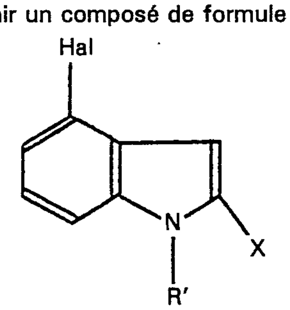

(III)

dans laquelle R' représente un radical alkyle renfermant de 1 à 8 atomes de carbone ou un radical aralkyle renfermant de 7 à 12 atomes de carbone, que l'on transforme en dérivé organo-magnésien, pour obtenir un composé de formule (IV):

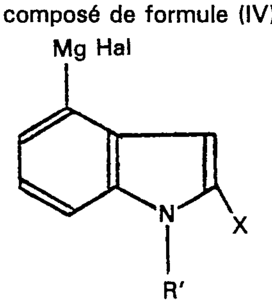

(IV)

que l'on condense avec la N-benzyl 3-pipéridone, pour obtenir un composé de formule ($I_A$):

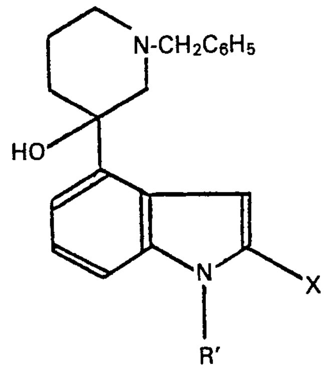

($I_A$)

puis, si désiré,

— *ou bien* soumet le composé de formule ($I_A$), à l'action d'un agent de déshydratation, pour obtenir un composé de formule ($I_B$):

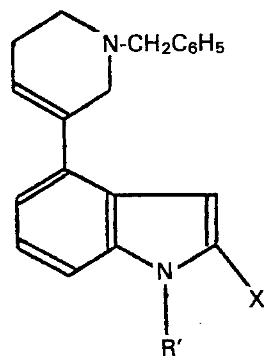

($I_B$)

que l'on soumet, si désiré, dans le cas où R' représente un radical benzyle, à l'action d'un agent de clivage sélectif du groupement benzyle porté par le noyau de l'indole, pour obtenir un composé de formule ($I_C$):

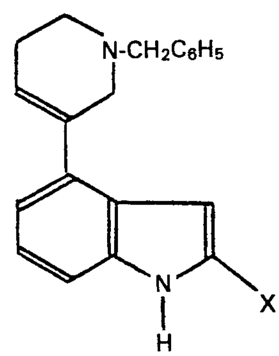

($I_C$)

que l'on soumet, si désiré, à l'action d'un agent de clivage du groupement benzyle porté par le noyau tétrahydropyridinyle, pour obtenir le composé de formule ($I_D$):

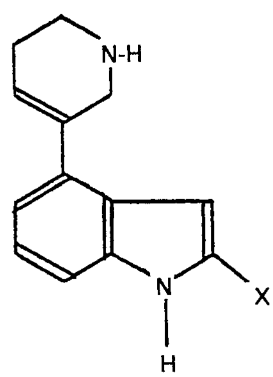

($I_D$)

— *ou bien* soumet le composé de formule ($I_A$), à l'action d'un agent de clivage du groupement benzyle porté par l'azote du groupement pipéridinyle, pour obtenir un composé de formule ($I_E$):

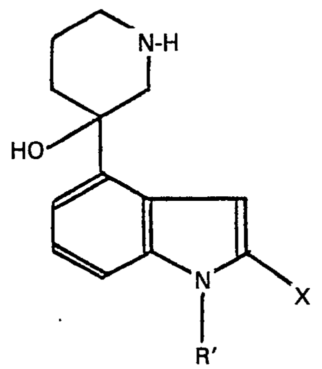

($I_E$)

que l'on soumet, si désiré, à l'action d'un agent de déshydratation, pour obtenir un composé de formule ($I_F$):

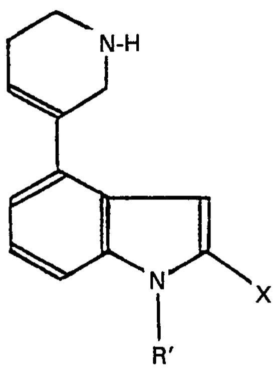

($I_F$)

— *ou bien,* dans le cas où R' représente un groupement benzyle, soumet le composé de formule ($I_A$), à l'action d'un agent de clivage sélectif du groupement benzyle porté par le noyau de l'indole, pour obtenir un composé de formule ($I_G$):

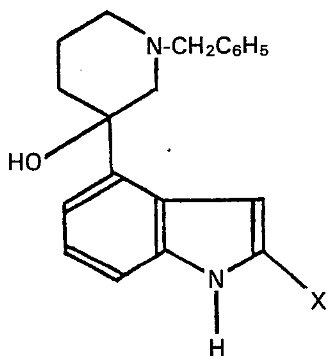

($I_G$)

que l'on soumet, si désiré, à l'action d'un agent de clivage du groupement benzyle porté par le noyau pipéridinyle, pour obtenir un composé de formule ($I_H$):

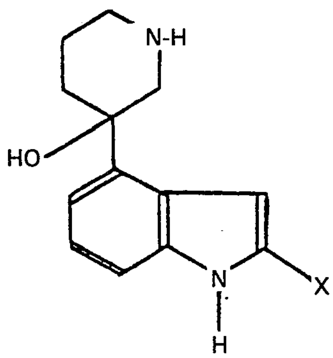

($I_H$)

puis soumet, si désiré, chacun des composés obtenus répondant à la formule (I) dans laquelle Z représente un atome d'hydrogène à l'action d'un agent capable d'introduire un radical Z', Z' ayant la même signification que Z, enoncée précédemment, à l'exception d'hydrogène, pour obtenir les composés de formule (I) correspondants dans lesquels l'atome d'azote du noyau pipéridinyle porte un radical Z' et, si désiré,

— soit l'on soumet chacun des composés obtenus répondant à la formule (I) dans laquelle b représente un radical hydroxyle à l'action d'un agent d'éthérification, pour obtenir les composés de formule (I) correspondants dans laquelle b représente un radical alkoxy renfermant de 1 à 8 atomes de carbone,

— soit l'on soumet chacun des composés obtenus répondant à la formule (I) dans laquelle b représente un radical hydroxyle à l'action d'un agent de déshydratation, pour obtenir les composés de formule (I) correspondants dans laquelle a et b forment ensemble une double liaison carbone-carbone,

— soit l'on soumet chacun des composés obtenus répondant à la formule (I) dans laquelle b représente un radical hydroxyle à l'action d'un agent de clivage du groupement hydroxyle, pour obtenir les composés de formule (I) correspondants dans laquelle a et b représentent chacun un atome d'hydrogène et, si désiré, soumet chacun des composés de formule (I) ainsi obtenus à l'action d'un agent d'halogénation susceptible d'introduire un atome d'halogène Hal en position 3 de l'indole, pour obtenir le composé de formule (I) correspondant dans lequel Y représente un atome d'halogène, et, si désiré, soumet chacun des composés de formule (I) obtenus précédemment à l'action d'un acide pour en former le sel.

2. Procédé selon la revendication 1, caractérisé en ce que les différentes étapes facultatives sont réalisées dans quelqu'ordre que ce soit.

3. Procédé selon la revendication 1 ou 2, caractérisé en ce que l'on utilise au départ un composé de formule (II) dans laquelle X représente un atome d'hydrogène.

4. Procédé selon la revendication 1 ou 2, caractérisé en ce que l'on prépare des composés de formule (I), dans laquelle Y représente un atome d'hydrogène.

5. Procédé selon l'une quelconque des revendications 1 à 4, caractérisé en ce que l'on prépare des composés de formule (I), dans laquelle Z représente un atome d'hydrogène.

6. Procédé selon l'une quelconque des revendications 1 à 4, caractérisé en ce que l'on prépare des composés de formule (I), dans laquelle Z représente un radical alkyle renfermant de 1 à 4 atomes de carbone ou un radical aralkyle renfermant de 7 à 12 atomes de carbone.

7. Procédé selon l'une quelconque des revendications 1 à 6, caractérisé en ce que l'on prépare des composés de formule (I), dans laquelle a et b forment ensemble une double liaison carbone-carbone.

8. Procédé selon l'une quelconque des revendications 1 à 6, caractérisé en ce que l'on prépare des composés de formule (I), dans laquelle a et b représentent chacun un atome d'hydrogène.

9. Procédé selon l'une quelconque des revendications 1 à 6, caractérisé en ce que l'on prépare des composés de formule (I), dans laquelle a représente un atome d'hydrogène et b un radical hydroxyle ou méthoxy.

10. Procédé selon la revendication 1 ou 2, caractérisé en ce que l'on prépare le 4-(pipéridin-3-yl) 1H-indole ainsi que ses sels d'addition avec les acides.

11. Procédé selon la revendication 1 ou 2, caractérisé en ce que l'on prépare le 4-(1,2,5,6-tétrahydropyridin-3-yl) 1H-indole ainsi que ses sels d'addition avec les acides.

12. Procédé selon la revendication 1 ou 2, caractérisé en ce que l'on prépare le 4-(1-propyl pipéridin-3-yl) 1H-indole, ainsi que ses sels d'addition avec les acides.

**Patentansprüche für die Vertragsstaaten:**
**BE, CH, LI, DE, FR, GB, IT, LU, NL, SE**

1. Verbindung der Formel I

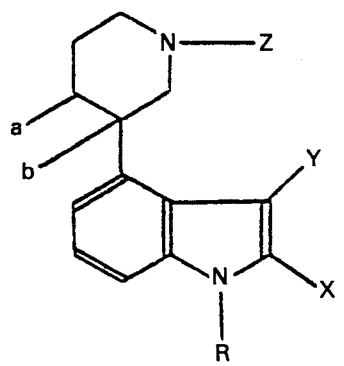

(I)

worin R ein Wasserstoffatom, einen Alkylrest mit 1 bis 8 Kohlenstoffatomen oder einen Aralkylrest mit 7 bis 12 Kohlenstoffatomen bedeutet, X ein Wasserstoffatom oder einen Alkylrest mit 1 bis 8 Kohlenstoffatomen bedeutet, Y ein Wasserstoffatom oder ein Halogenatom bedeutet, Z ein Wasserstoffatom, einen Alkyl- oder Hydroxylalkylrest mit 1 bis 8 Kohlenstoffatomen, einen Aryloxyalkylrest mit 7 bis 12 Kohlenstoffatomen, einen Aralkylrest mit 7 bis 12 Kohlenstoffatomen, gegebenenfalls substituiert durch ein oder mehrere Halogenatome, durch ein oder mehrere Alkoxyreste mit 1 bis 4 Kohlenstoffatomen, durch ein oder mehrere Alkylreste mit 1 bis 4 Kohlenstoffatomen oder durch einen oder mehrere Reste OH, $CF_3$, $OCF_3$, $NO_2$ oder $NH_2$, bedeutet oder Z einen Cycloalkylalkylrest mit 4 bis 12 Kohlenstoffatomen, einen Alkenylrest mit 3 bis 8 Kohlenstoffatomen oder einen Alkinylrest mit 3 bis 8 Kohlenstoffatomen bedeutet und

entweder a und b jeweils ein Wasserstoffatom bedeuten

oder a ein Wasserstoffatom bedeutet und b einen Hydroxylrest oder einen Alkoxyrest mit 1 bis 8 Kohlenstoffatomen bedeutet

oder a und b gemeinsam eine Kohlenstoff-Kohlenstoff-Doppelbindung bilden

sowie die Additionssalze der Verbindungen der Formel I mit Säuren.

2. Die Verbindungen der Formel I gemäss Anspruch 1, bei denen Y ein Wasserstoffatom bedeutet, sowie deren Additionssalze mit Säuren.

3. Die Verbindungen der Formel I gemäss Anspruch 1 oder 2, bei denen X ein Wasserstoffatom bedeutet, sowie deren Additionssalze mit Säuren.

4. Die Verbindungen der Formel I gemäss Anspruch 1, 2 oder 3, bei denen Z ein Wasserstoffatom bedeutet, sowie deren Additionssalze mit Säuren.

5. Die Verbindungen der Formel I gemäss einem der Ansprüche 1, 2 oder 3, bei denen Z einen Alkylrest mit 1 bis 4 Kohlenstoffatomen oder einen Aralkylrest mit 7 bis 12 Kohlenstoffatomen bedeutet, sowie deren Additionssalze mit Säuren.

6. Die Verbindungen der Formel I gemäss einem der Ansprüche 1 bis 5, bei denen a und b gemeinsam eine Kohlenstoff-Kohlenstoff-Doppelbindung bilden sowie deren Additionssalze mit Säuren.

7. Die Verbindungen der Formel I gemäss einem der Ansprüche 1 bis 5, bei denen a und b jeweils ein Wasserstoffatom bedeuten, sowie deren Additionssalze mit Säuren.

8. Die Verbindungen der Formel I gemäss einem der Ansprüche 1 bis 5, bei denen a ein Wasserstoffatom bedeutet und b einen Hydroxylrest oder einen Methoxyrest darstellt, sowie deren Additionssalze mit Säuren.

9. 4-(Piperidin-3-yl)-1H-indol sowie dessen Additionssalze mit Säuren.

10. 4-(1,2,5,6-Tetrahydropyridin-3-yl)-1H-indol sowie dessen Additionssalze mit Säuren.

11. Verfahren zur Herstellung der Verbindungen der Formel I gemäss einem der Ansprüche 1 bis 10, dadurch gekennzeichnet, dass man eine Verbindung der Formel II

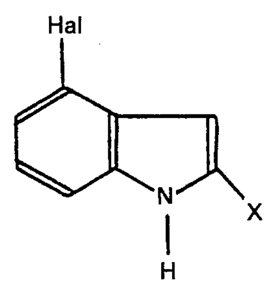

(II)

worin X ein Wasserstoffatom oder einen Alkylrest mit 1 bis 8 Kohlenstoffatomen bedeutet und Hal ein Halogenatom darstellt, der Einwirkung eines Alkylierungs- oder Aralkylierungsmittels unterzieht, um eine Verbindung der Formel III

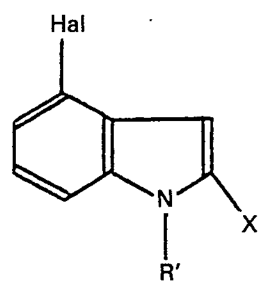

(III)

zu erhalten, worin R' einen Alkylrest mit 1 bis 8 Kohlenstoffatomen oder einen Aralkylrest mit 7 bis 12 Kohlenstoffatomen bedeutet, die man in ein Organomagnesiumderivat überführt, um eine Verbindung der Formel IV

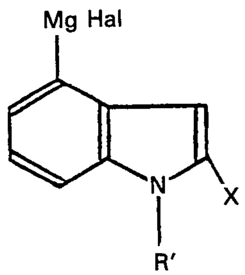

(IV)

zu erhalten, die man mit N-Benzyl-3-piperidon kondensiert, um eine Verbindung der Formel $I_A$

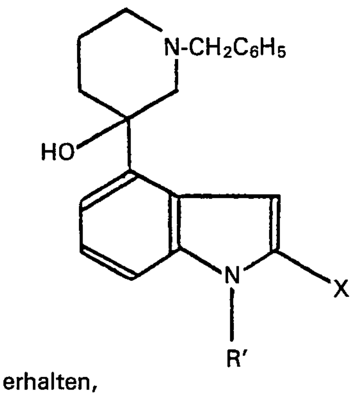

($I_A$)

zu erhalten,
danach gewünschtenfalls
*entweder* die Verbindung der Formel $I_A$ der Einwirkung eines Dehydratationsmittels unterzieht, um eine Verbindung der Formel $I_B$

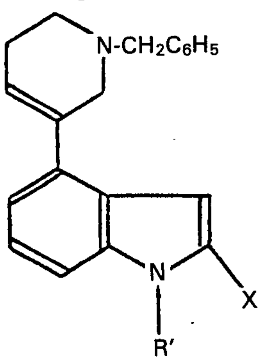

($I_B$)

zu erhalten, die man gewünschtenfalls, wenn R' einen Benzylrest bedeutet, der Einwirkung eines selektiven Abspaltungsmittels für die von dem Indolkern getragene Benzylgruppe unterzieht, um eine Verbindung der Formel $I_C$

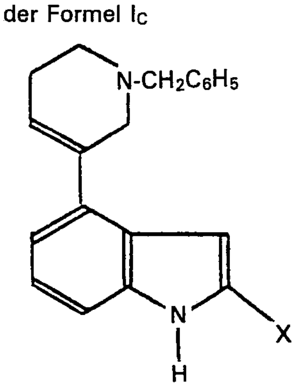

($I_C$)

zu erhalten, die man gewünschtenfalls der Einwirkung eines Abspaltungsmittels für die von dem Tetrahydropyridinylkern getragene Benzylgruppe unterzieht, um die Verbindung der Formel $I_D$

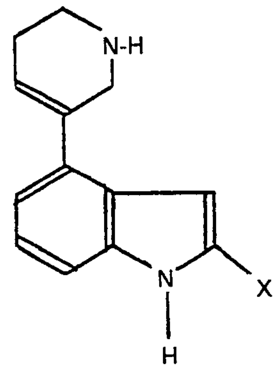

($I_D$)

zu erhalten
*oder* die Verbindung der Formel $I_A$ der Einwirkung eines Abspaltungsmittels für die von dem Stickstoffatom der Piperidinylgruppe getragene Benzylgruppe unterzieht, um eine Verbindung der Formel $I_E$

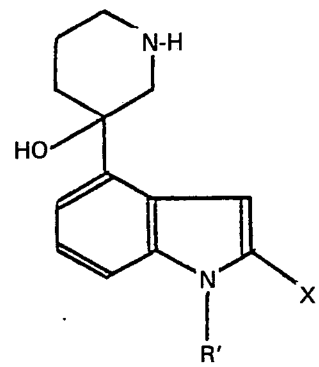

($I_E$)

zu erhalten, die man gewünschtenfalls der Einwirkung eines Dehydratationsmittels unterzieht, um eine Verbindung der Formel $I_F$

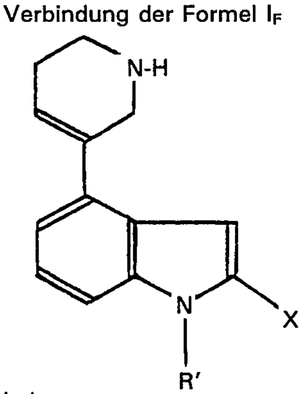

($I_F$)

zu erhalten
*oder* wenn R' eine Benzylgruppe bedeutet, die Verbindung der Formel $I_A$ der Einwirkung eines selektiven Abspaltungsmittels für die von dem Indolkern getragene Benzylgruppe unterzieht, um eine Verbindung der Formel $I_G$

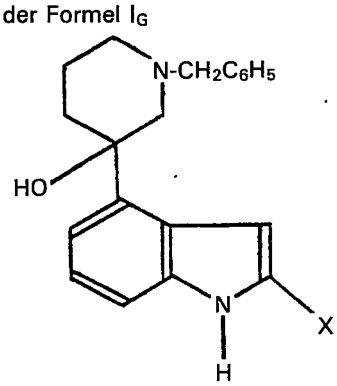

($I_G$)

zu erhalten, die man gewünschtenfalls der Einwirkung eines Abspaltungsmittels für die von dem Piperidinylkern getragene Benzylgruppe unterzieht, um eine Verbindung der Formel $I_H$

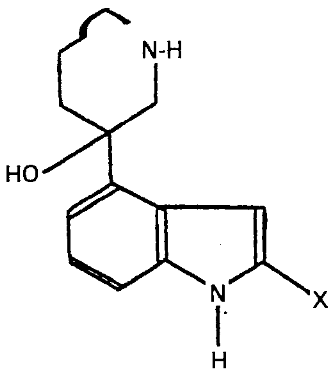

($I_H$)

zu erhalten

danach gewünschtenfalls eine jede der erhaltenen Verbindungen der Formel I, worin z ein Wasserstoffatom bedeutet, der Einwirkung eines Mittels unterzieht, das befähigt ist, einen Rest Z' einzuführen, wobei Z' die gleiche Bedeutung wie Z gemäss Anspruch 1 mit der Ausnahme von Wasserstoff besitzt, um die entsprechenden Verbindungen der Formel I zu erhalten, worin das Stickstoffatom des Piperidinylkerns einen Rest Z' trägt und gewünschtenfalls

entweder eine jede der erhaltenen Verbindungen der Formel I, worin b einen Hydroxylrest bedeutet, der Einwirkung eines Verätherungsmittels unterzieht, um die entsprechenden Verbindungen der Formel I zu erhalten, worin b einen Alkoxyrest mit 1 bis 8 Kohlenstoffatomen bedeutet,

oder eine jede der erhaltenen Verbindungen der Formel I, worin b einen Hydroxylrest bedeutet, der Einwirkung eines Dehydratationsmittels unterzieht, um die entsprechenden Verbindungen der Formel I zu erhalten, worin a und b gemeinsam eine Kohlenstoff-Kohlenstoff-Doppelbindung bilden,

oder eine jede der erhaltenen Verbindungen der Formel I, worin b einen Hydroxylrest bedeutet, der Einwirkung eines Abspaltungsmittels für die Hydroxylgruppe unterzieht, um die entsprechenden Verbindungen der Formel I zu erhalten, worin a und b jeweils ein Wasserstoffatom bedeuten und gewünschtenfalls eine jede der so erhaltenen Verbindungen der Formel I der Einwirkung eines Halogenierungsmittels unterzieht, das befähigt ist, ein Halogenatom Hal in 3-Stellung des Indols einzuführen, um die entsprechende Verbindung der Formel I zu erhalten, worin Y ein Halogenatom bedeutet und gewünschtenfalls eine der vorstehend erhaltenen Verbindungen der Formel I der Einwirkung einer Säure unterzieht, um hieraus das Salz zu bilden.

12. Verfahren gemäss Anspruch 11, dadurch gekennzeichnet, dass die verschiedenen fakultativen Stufen in beliebiger Reihenfolge durchgeführt werden.

13. Als Arzneimittel die Verbindungen der Formel I gemäss einem der Ansprüche 1 bis 8 sowie deren Additionssalze mit pharmazeutisch verträglichen Säuren.

14. Als Arzneimittel die Verbindungen der Formel I gemäss einem der Ansprüche 9 oder 10 sowie deren Additionssalze mit pharmazeutisch verträglichen Säuren.

15. Als Arzneimittel das 4-(piperidin-3-yl)-1H-indolhydrochlorid.

16. Pharmazeutische Zusammensetzungen, dadurch gekennzeichnet, dass sie als Wirkstoff zumindest eines der Arzneimittel gemäss einem der Ansprüche 13, 14 oder 15 enthalten.

**Patentansprüche für den Vertragsstaat: AT**

1. Verfahren zur Herstellung der Verbindungen der Formel I

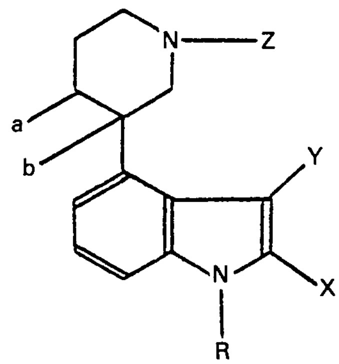

(I)

worin R ein Wasserstoffatom, einen Alkylrest mit 1 bis 8 Kohlenstoffatomen oder einen Aralkylrest mit 7 bis 12 Kohlenstoffatomen bedeutet, X ein Wasserstoffatom oder einen Alkylrest mit 1 bis 8 Kohlenstoffatomen bedeutet, Y ein Wasserstoffatom oder ein Halogenatom bedeutet, Z ein Wasserstoffatom, einen Alkyl- oder Hydroxylalkylrest mit 1 bis 8 Kohlenstoffatomen, einen Aryloxyalkylrest mit 7 bis 12 Kohlenstoffatomen, einen Aralkylrest mit 7 bis 12 Kohlenstoffatomen, gegebenenfalls substituiert durch ein oder mehrere Halogenatome, durch ein oder mehrere Alkoxyreste mit 1 bis 4 Kohlenstoffatomen, durch ein oder mehrere Alkylreste mit 1 bis 4 Kohlenstoffatomen oder durch einen oder mehrere Reste OH, $CF_3$, $OCF_3$, $NO_2$ oder $NH_2$, bedeutet oder Z einen Cycloalkylalkylrest mit 4 bis 12 Kohlenstoffatomen, einen Alkenylrest mit 3 bis 8 Kohlenstoffatomen oder einen Alkinylrest mit 3 bis 8 Kohlenstoffatomen bedeutet und

entweder a und b jeweils ein Wasserstoffatom bebedeuten

oder a ein Wasserstoffatom bedeutet und b einen Hydroxylrest oder einen Alkoxyrest mit 1 bis 8 Kohlenstoffatomen bedeutet

oder a und b gemeinsam eine Kohlenstoff-Kohlenstoff-Doppelbindung bilden

sowie von deren Additionssalzen mit Säuren, dadurch gekennzeichnet, dass man eine Verbindung der Formel II

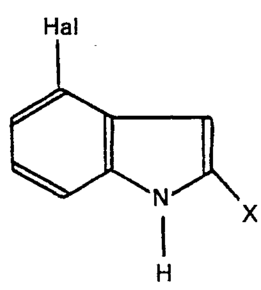

(II)

worin X ein Wasserstoffatom oder einen Alkylrest mit 1 bis 8 Kohlenstoffatomen bedeutet und Hal ein Halogenatom darstellt, der Einwirkung eines Alkylierungs- oder Aralkylierungsmittels unterzieht, um eine Verbindung der Formel III

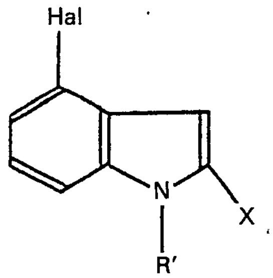

(III)

zu erhalten, worin R' einen Alkylrest mit 1 bis 8 Kohlenstoffatomen oder einen Aralkylrest mit 7 bis 12 Kohlenstoffatomen bedeutet, die man in ein Organomagnesiumderivat überführt, um eine Verbindung der Formel IV

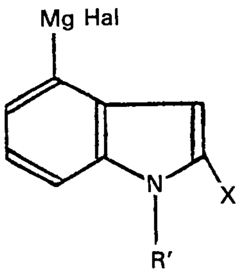

(IV)

zu erhalten, die man mit N-Benzyl-3-piperidon kondensiert, um eine Verbindung der Formel $I_A$

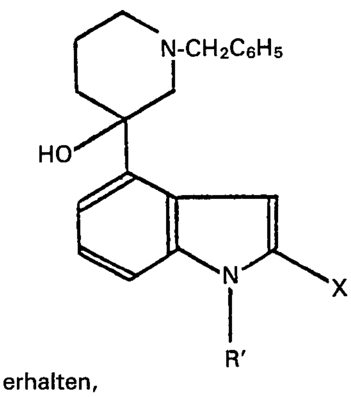

$(I_A)$

zu erhalten,
danach gewünschtenfalls
*entweder* die Verbindung der Formel $I_A$ der Einwirkung eines Dehydratationsmittels unterzieht, um eine Verbindung der Formel $I_B$

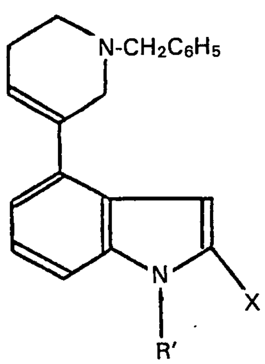

$(I_B)$

zu erhalten, die man gewünschtenfalls, wenn R' einen Benzylrest bedeutet, der Einwirkung eines selektiven Abspaltungsmittels für die von dem Indolkern getragene Benzylgruppe unterzieht, um eine Verbindung der Formel $I_C$

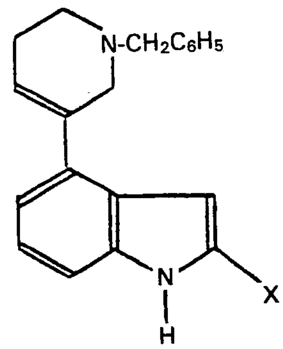

$(I_C)$

zu erhalten, die man gewünschtenfalls der Einwirkung eines Abspaltungsmittels für die von dem Tetrahydropyridinylkern getragene Benzylgruppe unterzieht, um die Verbindung der Formel $I_D$

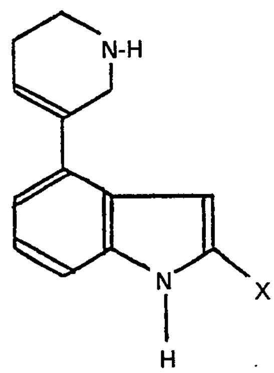

$(I_D)$

zu erhalten
*oder* die Verbindung der Formel $I_A$ der Einwirkung eines Abspaltungsmittels für die von dem Stickstoffatom der Piperidinylgruppe getragene Benzylgruppe unterzieht, um eine Verbindung der Formel $I_E$

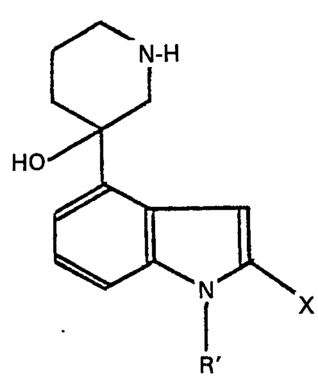

$(I_E)$

zu erhalten, die man gewünschtenfalls der Einwirkung eines Dehydratationsmittels unterzieht, um eine Verbindung der Formel $I_F$

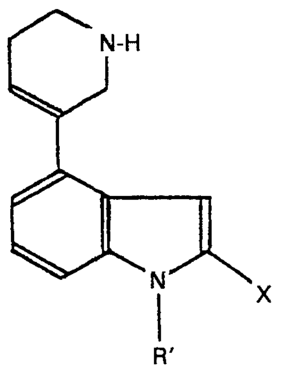

$(I_F)$

zu erhalten

*oder* wenn R' eine Benzylgruppe bedeutet, die Verbindung der Formel $I_A$ der Einwirkung eines selektiven Abspaltungsmittels für die von dem Indolkern getragene Benzylgruppe unterzieht, um eine Verbindung der Formel $I_G$

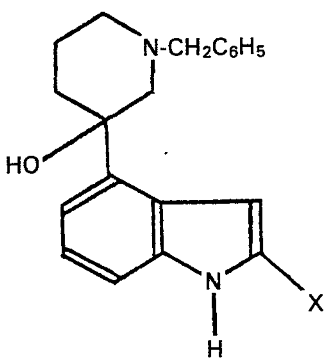

$(I_G)$

zu erhalten, die man gewünschtenfalls der Einwirkung eines Abspaltungsmittels für die von dem Piperidinylkern getragene Benzylgruppe unterzieht, um eine Verbindung der Formel $I_H$

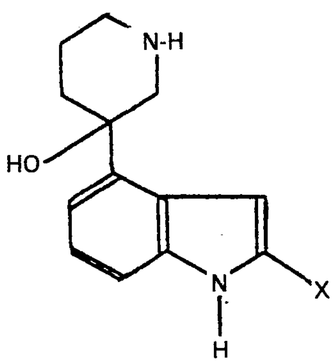

$(I_H)$

zu erhalten

danach gewünschtenfalls eine jede der erhaltenen Verbindungen der Formel I, worin z ein Wasserstoffatom bedeutet, der Einwirkung eines Mittels unterzieht, das befähigt ist, einen Rest Z' einzuführen, wobei Z' die gleiche Bedeutung wie vorstehend für Z angegeben mit der Ausnahme von Wasserstoff besitzt, um die entsprechenden Verbindungen der Formel I zu erhalten, worin das Stickstoffatom des Piperidinylkerns einen Rest Z' trägt und gewünschtenfalls

entweder eine jede der erhaltenen Verbindungen der Formel I, worin b einen Hydroxylrest bedeutet, der Einwirkung eines Verätherungsmittels unterzieht, um die entsprechenden Verbindungen der Formel I zu erhalten, worin b einen Alkoxyrest mit 1 bis 8 Kohlenstoffatomen bedeutet,

oder eine jede der erhaltenen Verbindungen der Formel I, worin b einen Hydroxylrest bedeutet, der Einwirkung eines Dehydratationsmittels unterzieht, um die entsprechenden Verbindungen der Formel I zu erhalten, worin a und b gemeinsam eine Kohlenstoff-Kohlenstoff-Doppelbindung bilden,

oder eine jede der erhaltenen Verbindungen der Formel I, worin b einen Hydroxylrest bedeutet, der Einwirkung eines Abspaltungsmittels für die Hydroxylgruppe unterzieht, um die entsprechenden Verbindungen der Formel I zu erhalten, worin a und b jeweils ein Wasserstoffatom bedeuten und gewünschtenfalls eine jede der so erhaltenen Verbindungen der Formel I der Einwirkung eines Halogenierungsmittels unterzieht, das befähigt ist, ein Halogenatom Hal in 3-Stellung des Indols einzuführen, um die entsprechende Verbindung der Formel I zu erhalten, worin Y ein Halogenatom bedeutet und gewünschtenfalls eine der vorstehend erhaltenen Verbindungen der Formel I der Einwirkung einer Säure unterzieht, um hieraus das Salz zu bilden.

2. Verfahren gemäss Anspuch 1, dadurch gekennzeichnet, dass die verschiedenen fakultativen Stufen in beliebiger Reihenfolge durchgeführt werden.

3. Verfahren gemäss Anspruch 1 oder 2, dadurch gekennzeichnet, dass man von einer Verbindung der Formel II ausgeht, worin X ein Wasserstoffatom bedeutet.

4. Verfahren gemäss Anspruch 1 oder 2, dadurch gekennzeichnet, dass man Verbindungen der Formel I herstellt, worin Y ein Wasserstoffatom bedeutet.

5. Verfahren gemäss einem der Ansprüche 1 bis 4, dadurch gekennzeichnet, dass man Verbindungen der Formel I herstellt, worin Z ein Wasserstoffatom bedeutet.

6. Verfahren gemäss einem der Ansprüche 1 bis 4, dadurch gekennzeichnet, dass man Verbindungen der Formel I herstellt, worin Z einen Alkylrest mit 1 bis 4 Kohlenstoffatomen oder einen Aralkylrest mit 7 bis 12 Kohlenstoffatomen bedeutet.

7. Verfahren gemässeinem der Ansprüche 1 bis 6, dadurch gekennzeichnet, dass man Verbindungen der Formel I herstellt, worin a und b gemeinsam eine Kohlenstoff-Kohlenstoff-Doppelbindung bilden.

8. Verfahren gemäss einem der Ansprüche 1 bis 6, dadurch gekennzeichnet, dass man Verbindungen der Formel I herstellt, worin a und b jeweils ein Wasserstoffatom bedeuten.

9. Verfahren gemäss einem der Ansprüche 1 bis 6, dadurch gekennzeichnet, dass man Verbindungen der Formel I herstellt, worin a ein Wasserstoffatom und b einen Hydroxyl- oder Methoxyrest bedeutet.

10. Verfahren gemäss Anspruch 1 oder 2, dadurch gekennzeichnet, dass man 4-(Piperidin-3-yl)-1H-indol sowie dessen Additionssalze mit Säuren herstellt.

11. Verfahren gemäss Anspruch 1 oder 2, dadurch gekennzeichnet, dass man 4-(1,2,5,6-Tetrahydropyridin-3-yl)-1H-indol sowie dessen Additionssalze mit Säuren herstellt.

12. Verfahren gemäss Anspruch 1 oder 2, dadurch gekennzeichnet, dass man 4-(1-Propylpiperidin-3-yl)-1H-indol sowie dessen Additionssalze mit Säuren herstellt.

**Claims for the Contracting States:**
**BE, CH, LI, DE, FR, GB, IT, LU, NL, SE**

1. Compounds with the formula (I):

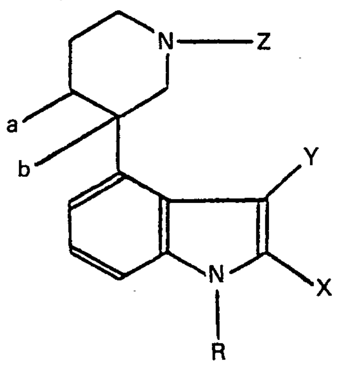

(I)

in which:

— R represents a hydrogen atom, an alkyl radical containing from 1 to 8 carbon atoms or an aralkyl radical containing from 7 to 12 carbon atoms.

— X represents a hydrogen atom or an alkyl radical containing from 1 to 8 carbon atoms,

— Y represents a hydrogen atom or a halogen atom,

— Z represents a hydrogen atom, an alkyl or hydroalkyl radical containing from 1 to 8 carbon atoms, an aryloxyalkyl radical containing from 7 to 12 carbon atoms, an aralkyl radical containing from 7 to 12 carbon atoms possibly substituted by one or more halogen atoms, by one or more alkoxy radicals containing from 1 to 4 carbon atoms, by one or more alkyl radicals containing from 1 to 4 carbon atoms, or by one or more radicals OH, $CF_3$, $OCF_3$, $NO_2$ or $NH_2$ or Z represents a cycloalkyl-alkyl radical containing from 4 to 12 carbon atoms, an alkenyl radical containing from 3 to 8 carbon atoms or an alkynyl radical containing from 3 to 8 carbon atoms and,

— either a and b each represent a hydrogen atom,

— or a represents a hydrogen atom and b represents a hydroxyl radical or an alkoxy radical containing from 1 to 8 carbon atoms,

— or a and b together form a double carbon-carbon bond as well as the salts of addition with acids of the compounds with the formula (I).

2. Compounds with the formula (I) as defined in claim 1, for which Y represents a hydrogen atom, as well as their salts of addition with acids.

3. Compounds with the formula (I) as defined in claim 1 or 2, for which X represents a hydrogen atom, as well as their salts of addition with acids.

4. Compounds with the formula (I) as defined in claim 1, 2, or 3, for which Z represents a hydrogen atom, as well as their salts of addition with acids.

5. Compounds with the formula (I) as defined in any one of the claims 1, 2 or 3, for which Z represents an alkyl radical containing from 1 to 4 carbon atoms or an aralkyl containing from 7 to 12 carbon atoms, as well as their salts of addition with acids.

6. Compounds with the formula (I) as defined at any one of claims 1 to 5, for which a and b together form a double carbon-carbon bond, as well as their salts of addition with acids.

7. Compounds with the formula (I) as defined in any one of claims 1 to 5 for which a and b each represents a hydrogen atom, as well as their salts of addition with acids.

8. Compounds with the formula (I) as defined at any one of claims 1 to 5 for which a represents a hydrogen atom and b represents a hydroxyl radical or a methoxy radical as well as their salts of addition with acids.

9. The 4-(piperidin-3-yl) 1H-indole as well as its salts of addition with acids.

10. The 4-(1,2,5,6-tetrahydropyridin-3-yl) 1H-indole as well as its salts of addition with acids.

11. Preparation process for the compounds with the formula (I) as defined in any of the claims 1 to 10 characterised in that a compound with the formula (II)

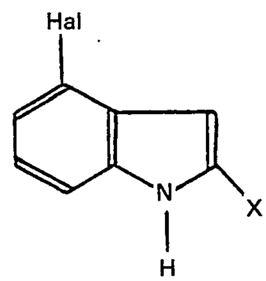

(II)

in which X represents a hydrogen atom or an alkyl radical containing from 1 to 8 carbon atoms and Hal represents a halogen atom is submitted to the action of an alkylation or aralkylation agent, so as to obtain a compound with the formula (III):

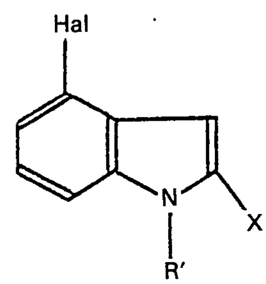

(III)

in which R' represents an alkyl radical containing from 1 to 8 carbon atoms or an aralkyl radical containing fromn 7 to 12 carbon atoms, which is converted into an organo-magnesium derivative, so as to obtain a compound with the formula (IV):

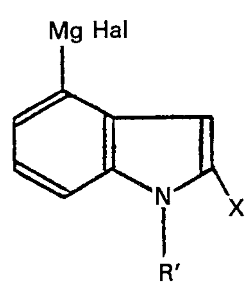

(IV)

which is condensed with N-benzyl 3-piperidone, so as to obtain a compound with the formula ($I_A$):

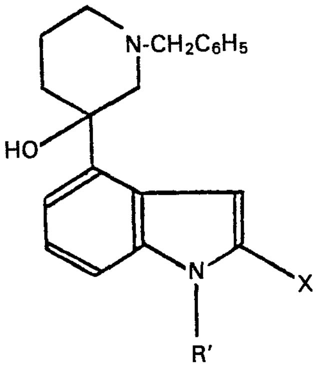

($I_A$)

then, if desired

— *either* the compound with the formula ($I_A$) is submitted to the action of a dehydratation agent, so as to obtain a compound with the formula ($I_B$):

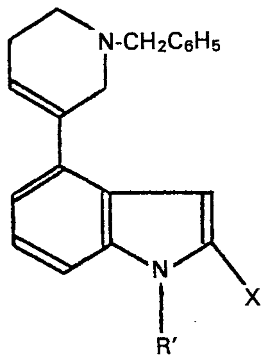

($I_B$)

which if desired, in the case where R' represents a benzyl radical is submitted to the action of a selective cleavage agent for the benzyl group carried by the nucleus of the indole, so as to obtain a compound with formula ($I_C$):

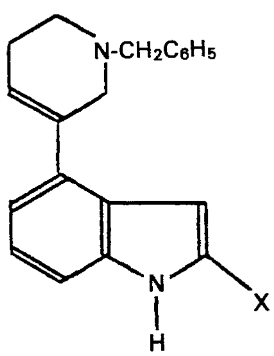

($I_C$)

which, if desired, is submitted to the action of a cleavage agent for the benzyl group carried by the tetrahydropyridinyl nucleus, so as to obtain the compound of the formula ($I_D$):

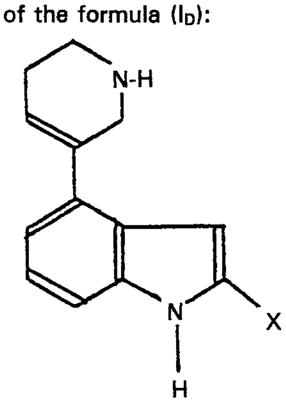

($I_D$)

— *or* the compound with the formula ($I_A$) is submitted to the action of a cleavage agent for the benzyl group carried by the nitrogen of the piperidinyl group, so as to obtain a compound with the formula ($I_E$):

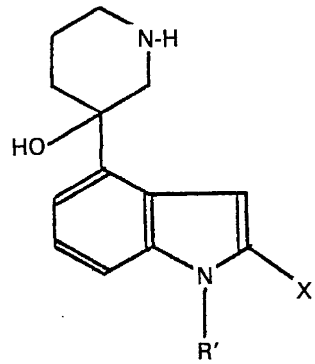

($I_E$)

which if desired is submitted to the action of a dehydratation agent, so as to obtain a compound with the formula ($I_F$):

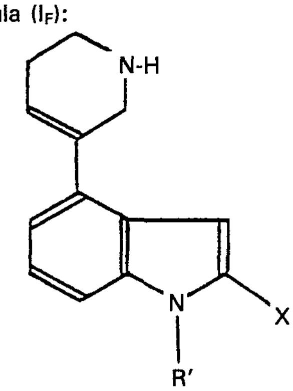

($I_F$)

— *or,* in the case where R' represents a benzyl group the compound with the formula ($I_A$) is submitted to the action of a selective cleavage agent for the benzyl group carried by the indole nucleus, so as to obtain a compound with the formula ($I_G$):

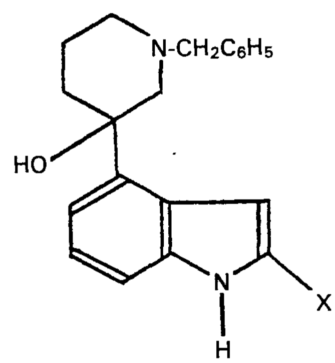

($I_G$)

which, if desired, is submitted to the action of a cleavage agent for the benzyl group carried by the piperidinyl nucleus so as to obtain a compound with the formula ($I_H$):

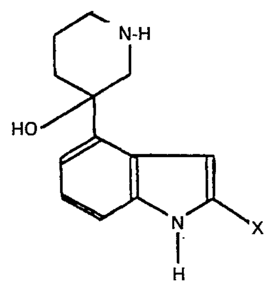

($I_H$)

then, if desired, each of the compounds obtained which respond to the formula (I) in which Z represents a hydrogen atom is submitted to the action of an agent able to introduce a radical Z', Z' having the same significance as Z, set out in claim 1, with the exception of hydrogen, so as to obtain the corresponding compounds with the formula (I) in which the nitrogen atom of the piperidinyl nucleus carries a radical Z' and, if desired,
— either each of the compounds obtained responding to the formula (I) in which b represents a hydroxyl radical is submitted to the action of an etherification agent, so as to obtain the corresponding compounds with the formula (I) in which b represents an alkoxy radical containing from 1 to 8 carbon atoms,
— or each of the compounds obtained responding to the formula (I) in which b represents a hydroxyl radical is submitted to the action of a dehydratation agent, so as to obtain the corresponding compounds with the formula (I) in which a and b together form a double carbon-carbon bond,
— or each of the compounds obtained responding to the formula (I) in which b represents a hydroxyl radical, is submitted to the action of a cleavage agent for the hydroxyl group, so as to obtain the corresponding compounds with the formula (I) in which a and b each represent a hydrogen atom and, if desired each of the compounds with the formula (I) so obtained is submitted to the action of a halogenation agent able to introduce a halogen atom Hal in position 3 of the indole, so as to obtain the corresponding compound with the formula (I) in which Y represents a halogen atom, and if desired, each of the compounds with the formula (I) previously obtained is submitted to the action of an acid so as to form its salt.

12. Process according to claim 11, characterised in that the different optional stages are carried out in any order whatsoever.

13. As medicaments, the compounds with the formula (I), as defined in any one of the claims 1 to 8 as well as their salts of addition with pharmaceutically acceptable acids.

14. As medicaments, the pharmaceutically acceptable compounds with the formula (I), as defined in one of the claims 9 or 10.

15. As medicament:
— 4-(piperidin-3-yl) 1H-indole hydrochloride.

16. The pharmaceutical compositions containing as active principle at least one of the medicaments as defined in claims 13, 14 or 15.

**Claims for the Contracting State: AT**

1. Process for the preparation of compounds with the formula (I):

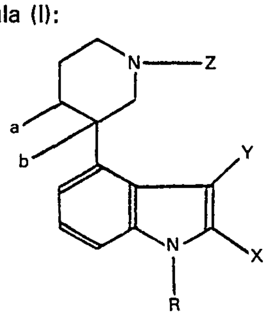

(I)

in which:
— R represents a hydrogen atom, an alkyl radical containing from 1 to 8 carbon atoms or an aralkyl radical containing from 7 to 12 carbon atoms,
— X represents a hydrogen atom or an alkyl radical containing from 1 to 8 carbon atoms,
— Y represents a hydrogen atom or a halogen atom,
— Z represents a hydrogen atom, an alkyl or hydroalkyl radical containing from 1 to 8 carbon atoms, an aryloxyalkyl radical containing from 7 to 12 carbon atoms, an aralkyl radical containing from 7 to 12 carbon atoms possibly substituted by one or more halogen atoms, by one or more alkoxy radicals containing from 1 to 4 carbon atoms, by one or more alkyl radicals containing from 1 to 4 carbon atoms, or by one or more radicals OH, $CF_3$, $OCF_3$, $NO_2$ or $NH_2$ or Z represents a cycloalkyl-alkyl radical containing from 4 to 12 carbon atoms, an alkenyl radical containing from 3 to 8 carbon atoms or an alkynyl radical containing from 3 to 8 carbon atoms and,
— either a and b each represent a hydrogen atom,
— or a represents a hydrogen atom and b represents a hydroxyl radical or an alkoxy radical containing from 1 to 8 carbon atoms,
— or a and b together form a double carbon-carbon bond as well as the salts of addition with acids characterised in that a compound with the formula (I).

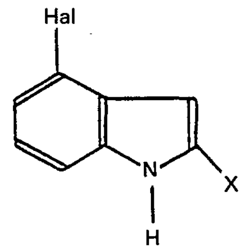

(II)

in which X represents a hydrogen atom or an alkyl radical containing from 1 to 8 carbon atoms and Hal represents a halogen atom is submitted to the action of an alkylation or aralkylation agent, so as to obtain a compound with the formula (III):

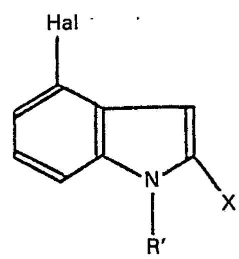

(III)

in which R' represents an alkyl radical containing from 1 to 8 carbon atoms or an aralkyl radical containing from 7 to 12 carbon atoms, which is converted into an organo-magnesium derivative, so as to obtain a compound with the formula (IV):

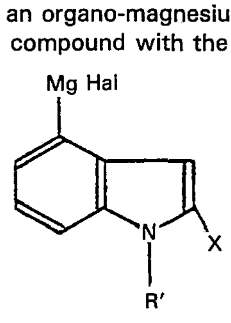

(IV)

which is condensed with N-benzyl 3-piperidone, so as to obtain a compound with the formula ($I_A$):

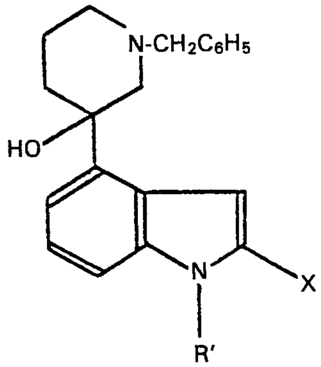

($I_A$)

then, if desired

— *either* the compound with the formula ($I_A$) is submitted to the action of a dehydratation agent, so as to obtain a compound with the formula ($I_B$):

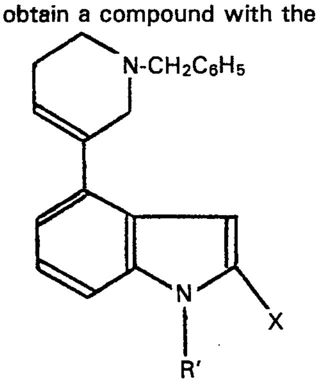

($I_B$)

which if desired, in the case where R' represents a benzyl radical is submitted to the action of a selective cleavage agent for the benzyl group carried by the nucleus of the indole, so as to obtain a compound with formula ($I_C$):

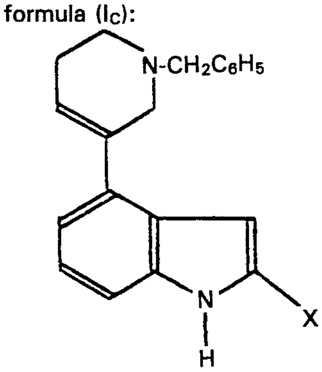

($I_C$)

which, if desired, is submitted to the action of a cleavage agent for the benzyl group carried by the tetrahydropyridinyl nucleus, so as to obtain the compound of the formula ($I_D$):

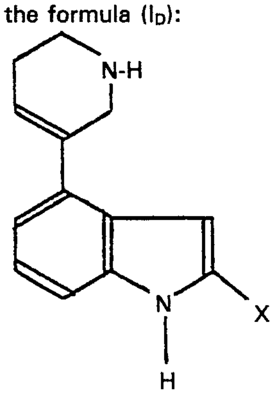

($I_D$)

— *or* the compound with the formula ($I_A$) is submitted to the action of a cleavage agent for the benzyl group carried by the nitrogen of the piperidinyl group, so as to obtain a compound with the formula ($I_E$):

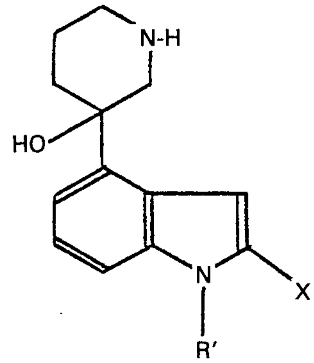

($I_E$)

which if desired is submitted to the action of a dehydratation agent, so as to obtain a compound with the formula ($I_F$):

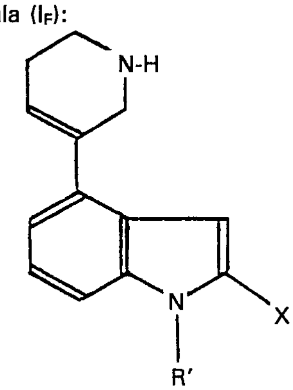

($I_F$)

— *or,* in the case where R' represents a benzyl group the compound with the formula ($I_A$) is submitted to the action of a selective cleavage agent for the benzyl group carried by the indole nucleus, so as to obtain a compound with the formula ($I_G$):

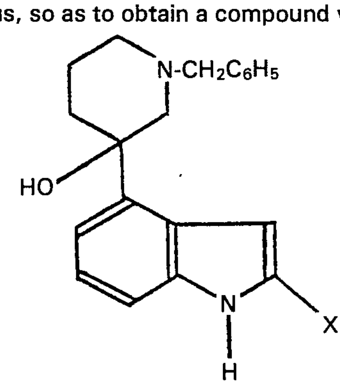

($I_G$)

which, if desired, is submitted to the action of a cleavage agent for the benzyl group carried by the piperidinyl nucleus so as to obtain a compound with the formula ($I_H$):

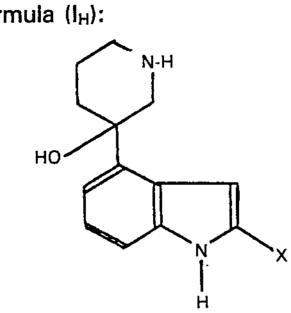

($I_H$)

then, if desired, each of the compounds obtained which respond to the formula (I) in which Z represents a hydrogen atom is submitted to the action of an agent able to introduce a radical Z', Z' having the same significance as Z, set out in claim 1, with the exception of hydrogen, so as to obtain the corresponding compounds with the formula (I) in which the nitrogen atom of the piperidinyl nucleus carries a radical Z' and, if desired,

— either each of the compounds obtained responding to the formula (I) in which b represents a hydroxyl radical is submitted to the action of an etherification agent, so as to obtain the corresponding compounds with the formula (I) in which b represents an alkoxy radical containing from 1 to 8 carbon atoms,

— or each of the compounds obtained responding to the formula (I) in which b represents a hydroxyl radical is submitted to the action of a dehydratation agent, so as to obtain the corresponding compounds with the formula (I) in which a and b together form a double carbon-carbon bond,

— or each of the compounds obtained responding to the formula (I) in which b represents a hydroxyl radical, is submitted to the action of a cleavage agent for the hydroxyl group, so as to obtain the corresponding compounds with the formula (I) in which a and b each represent a hydrogen atom and, if desired each of the compounds with the formula (I) so obtained is submitted to the action of a halogenation agent able to introduce a halogen atom Hal in position 3 of the indole, so as to obtain the corresponding compound with the formula (I) in which Y represents a halogen atom, and if desired, each of the compounds with the formula (I) previously obtained is submitted to the action of an acid so as to form its salt.

2. Process according to claim 1, characterised in that the different optional stages are carried out in any order whatsoever.

3. Process according to claim 1 or 2, characterised in that for starting a compound is used with the formula (II) in which X represents a hydrogen atom.

4. Process according to claim 1 or 2, characterised in that compounds with the formula (I) are prepared in which Y represents a hydrogen atom.

5. Process according to any one of claims 1 to 4, characterised in that compounds with the formula (I) are prepared in which Z represents a hydrogen atom.

6. Process according to any one of claims 1 to 4, characterised in that compounds with the formula (I) are prepared in which Z represents an alkyl radical containing from 1 to 4 carbon atoms or an aralkyl radical containing 7 to 12 carbon atoms.

7. Process according to any one of claims 1 to 6, characterised in that compounds with the formula (I) are prepared in which a and b together form a double carbon-carbon bond.

8. Process according to any one of claims 1 to 6, characterised in that compounds with the formula (I) are prepared in which a and b each represent a hydrogen atom.

9. Process according to any one of claims 1 to 6, characterised in that compounds with the formula (I) are prepared in which a represents a hydrogen atom and b a hydroxyl or methoxy radical.

10. Process according to claim 1 or 2, characterised in that 4-(piperidin-3-yl) 1H-indole is prepared, as well as its salts of addition with acids.

11. Process according to claim 1 or 2, characterised in that 4-(1,2,5,6-tetrahydropyridin-3-yl) 1H-indole is prepared, as well as its salts of addition with acids.

12. Process according to claim 1 or 2, characterised in that 4-(1-propyl piperidin-3-yl) 1H-indole is prepared, as well as its salts of addition with acids.